# DEMANDE DE BREVET EUROPEEN

(11) **EP 3 097 922 A1**
(43) Date de publication de la demande: **30.11.2016**
(21) Numéro de dépôt: 15305807.8
(22) Date de dépôt: 28.05.2015
(51) Int. Cl.: A61K 38/18, A61K 48/00, A61K 31/737, A61K 35/19, A61P 17/02, A61P 19/00, A61P 19/02, A61P 1/06, A61P 11/00, A61P 1/00

(54) **COMPOSITION POUR LE TRAITEMENT DES LESIONS TISSULAIRES**

(71) Demandeur: Barritault, Denis, 75001 Paris (FR); Organes Tissus Régénération Réparation Remplacement, 75001 Paris (FR)
(72) Inventeur: Barritault, Denis, 75001 Paris (FR); Organes Tissus Régénération Réparation Remplacement, 75001 Paris (FR)
(74) Mandataire: Novagraaf Technologies

(57) **Abrégé**

La présente invention se rapporte à une composition pharmaceutique comprenant un polymère biocompatible de formule générale (I) et (i) une cellule eucaryote, (ii) un extrait et/ou lysat plaquettaire ou (iii) au moins un facteur de croissance pour son application comme médicament pour la prévention et/ou le traitement de lésions tissulaires.

La présente invention se rapporte également à un kit pharmaceutique comprenant un polymère biocompatible de formule générale (I) et une cellule eucaryote pour la prévention et/ou le traitement de lésions tissulaires.

La présente invention se rapporte également utilisation d'une composition pharmaceutique comprenant un polymère biocompatible de formule générale (I) et (i) une cellule eucaryote, (ii) un extrait et/ou lysat plaquettaire ou (iii) au moins un facteur de croissance pour la fabrication d'un médicament pour le traitement de lésions tissulaires.

La présente invention trouve une application notamment dans les domaines pharmaceutiques et vétérinaire.

## Description

### Domaine technique

La présente invention se rapporte à une composition pharmaceutique pour son application comme médicament pour la prévention et/ou le traitement de lésions tissulaires.

La présente invention se rapporte également à un kit pharmaceutique pour la prévention et/ou le traitement de lésions tissulaires.

La présente invention se rapporte également utilisation d'une composition pharmaceutique pour la fabrication d'un médicament pour le traitement de lésions tissulaires.

La présente invention trouve une application notamment dans les domaines pharmaceutiques et vétérinaire.

Dans la description ci-dessous, les références entre parenthèses ( ) renvoient à la liste des références présentée à la fin du texte.

### Etat de la technique

L'implantation de cellules, de tissus ou d'organes à des fins thérapeutiques est un enjeu majeur de la médecine. De nombreux travaux étalés sur près d'un siècle ont démontré les bénéfices thérapeutiques de ces implantations dans des applications multiples et de nombreuses techniques ont permis de maitriser et d'améliorer la qualité des prélèvements, leur conservation et préservation, ou leur expansion avant de les implanter ou de les réintroduire chez le patient receveur. De même de nombreuses améliorations ont été apportées aux performances et propriétés des organes, tissus ou cellules qui sont utilisés dans des procédés, dispositifs et produits en vue d'apporter après une réintroduction chez le patient receveur un bénéfice thérapeutique. Enfin une meilleure connaissance et maitrise des réactions adverses parfois associées à cette réintroduction a aussi permis d'assurer un meilleur succès.

Toutefois, après administration des cellules, les rendements concernant leur intégration et/ou l'efficacité thérapeutique au vu du nombre de cellules administrées reste très faible, impliquant ainsi la nécessité de répétition du traitement et des coûts de traitement très importants. En outre, les faibles rendements et coûts importants limitent les pathologies et/ou les patients susceptibles d'être traités par ces procédés.

Il existe donc un réel besoin dans l'état de la technique de pouvoir encore améliorer ces procédés et/ou les outils biologiques utilisés afin d'optimiser leur utilisation et de pouvoir élargir le champ thérapeutique d'application.

Il existe dans l'état de la technique des composés susceptibles d'améliorer l'environnement tissulaire. Par exemple, les polymères nommés HBGFPP pour « Heparan Binding Growth Factors Protectors and Potentiators), ces HBGFPP étaient définis par leur propriété à protéger les facteurs de croissance présentant de l'affinité pour l'héparine (comme les FGF, TGFb et d'autres), contre des dégradations par les protéases ainsi que pour potentialiser l'activité de ces facteurs sans présenter d'activité anticoagulante significatives aux doses utilisées. Ces HBGFPP présentaient des propriétés surprenantes sur la réparation des lésions de tissus musculaires, nerveux, les tissus du tractus digestif ainsi qu'une activité sur la réaction inflammatoire, comme illustré notamment la demande internationale WO1995026739 ou dans le brevet américain US7998922. Ces polymères ont été également définis chimiquement et illustrés comme des agents de régénération tissulaires dans des modèles précliniques de lésion des tissus cutanées, osseux, de cornées et ischémies. Ils ont également démontré des effets anti-fibrotiques, des capacités uniques de protection contre les effets du stress oxydatif dans des modèles d'ischémie, des effets protecteurs contre le vieillissement et la neurodégénérescence et d'une manière générale ils favorisent des processus de régénération. Une forme particulière de HBGFPP, les RGTA, ont démontré, lors de leur utilisation une accélération et amélioration de la réparation de lésions tissulaires comme la peau, la cornée, l'os ou encore le muscle ; et éventuellement une diminution des traces cicatricielles et plus généralement des fibroses (voir revue Van Neck et al., Heparan Sulfate Proteoglycan Mimetics Promote Tissue Regeneration: An Overview chapter 4 in Tissue Regeneration - From Basic Biology to Clinical Application ISBN 978-953-51-0387-5, edited by Jamie Daviers). Les RGTA sont également connus comme pouvant protéger d'effets délétères dues aux irradiations- (Mangoni M et al.; Int. J. Radiation Oncology Biol. Phys. 2009,74,1242-1250), au stress oxydatif dans les cas de lésions provoqués par irradiations (Yue X-L et al.; Cell Death and Differentiation 2009, 1-12), au stress oxydatif lors d'ischémies (Desgranges et al.; FASEB J. 1999 Apr; 13(6):761-6.) ou également dans le cas du vieillissement tissulaire (Larramendy-Gozalo C, D,. et al J Gen Virol. 2007, 88:1062-7).

Dans un autre domaine thérapeutique, d'autres effets des RGTA ont été également observés, notamment dans le traitement de la douleur et de la démangeaison et pour lesquels l'homme de l'art ne pouvait prévoir un lien avec le processus de réparation ou la régénération tissulaire puisque l'effet était très rapide et donc indépendant du processus de réparation ou régénération beaucoup plus long à opérer.

Les RGTA ont été décrits et utilisés pour réparer les tissus lésés via notamment la protection des facteurs de croissance et de communications cellulaires présents sur le site lésionnel ainsi que le recrutement des cellules existantes chez le patient traité. Par exemple des publications ont montré un effet des RGTA sur la mobilisation des cellules endogènes souches comme des cellules pro génitrices hématopoïétiques, ou des cellules souches dérivés du sang (Albanes et al., Experimental Hematology 2009;37:1072-1083) ou sur la croissance, clonogénicité, la migration et/ou différenciation des cellules souches mésenchymateuses in vitro ou in vivo.

L'utilisation de RGTA comprenant de la benzylamine avec des cellules de la moelle osseuse a été envisagée dans la demande internationale WO 2003101201 pour le traitement des infarctus du myocarde pour augmenter la formation de la vascularisation de collatéraux. Toutefois, cette divulgation ne comprend aucune expérimentation, ni aucun résultats scientifiques. Ainsi, les éléments compris dans ce document ne permettent pas la reproduction des éléments décrits ni d'envisager un quelconque traitement. En particulier, le document Mullanghi et al., (Coronary; 22: 71) rapportent que le RGTA co-injecté avec les cellules mésenchymateuses prélevées le même jour de la moelle osseuse et directement injectées dans la zone infarcie après ligature de la coronaire descendante chez le Babouin n'a pas plus d'efficacité fonctionnelle que le RGTA injecté seul dans ce muscle, reproduisant ainsi les observations de Yamauchi H et al., FASEB J. 2000 (14): 2133-4, qui montrait que le RGTA seul avait une forte capacité d'amélioration de la récupération post infarctus du myocarde. La co-injection des cellules et du RGTA n'apporte donc aucun effet bénéfique supplémentaire concernant la récupération post infarctus du myocarde par rapport à l'utilisation du RGTA seul. En d'autres termes, aucun effet bénéfique supplémentaire n'a été observé/démontré en injectant des cellules et des RGTA.

La littérature fait également état de nombreuses études de thérapies cellulaires visant à implanter par différents méthodes des cellules de différentes provenances et natures afin de recoloniser des zones de tissus lésés ou aux fonctions altérées. De toutes ces études, il ressort une très grande difficulté à obtenir une survie des cellules implantées dans la zone visée, plus difficile encore est d'obtenir une colonisation de l'espace et plus encore une récupération dans la durée des fonctions de l'organe ou du tissu ayant été ainsi recolonisé. Ce problème reste encore un véritable challenge qui n'est pas encore résolu d'une manière satisfaisante.

Il existe donc un réel besoin de trouver un nouveau composé et/ou procédé susceptible d'améliorer le traitement de lésions tissulaires et/ou l'efficacité des thérapies cellulaires.

L'utilisation du RGTA mélangé à un biomatériau substitut osseux est décrite dans l'art antérieur (Billy et al., Patent US 2006/0257449). Ce document envisage également l'association de ce biomatériaux (tricalcique), avec des cellules souches non pas les RGTA, pour essayer de favoriser l'implantation et la colonisation du substitut osseux toutefois sans explication aucune, ni description quant à une éventuelle mise en oeuvre, étape etc. En outre, cette divulgation ne comprend aucune expérimentation, ni aucun résultats scientifiques. Ainsi, les éléments décrits dans ce document et son enseignement ne permettent pas la reproduction ni l'élaboration d'une quelconque association entre un biomatériau substitut osseux et des cellules, ni une quelconque utilisation d'une quelconque association.

De nombreuses pathologies, notamment dues au vieillissement, comprennent des phénomènes de dégénérescence tissulaire et/ou cellulaire, par exemple la maladie d'Alzheimer, la dégénérescence maculaire, et/ou provoqué par des phénomènes biologiques tel que la diminution de l'oxygénation des tissus/cellules etc.

Pour ces pathologies, et comme mentionné ci-dessus, il existe un grand besoin dans l'état de la technique de trouver des traitements efficaces, et/ou notamment d'améliorer les traitements existants, en particulier concernant les thérapies cellulaires.

Il existe également de nombreuses pathologies génétiques impliquant notamment de mauvais fonctionnement de tissus et/ou de cellules pour lesquels les traitements sont quasi inexistants ou peu efficace. Pour ces pathologies, une voie de traitement consiste dans la thérapie cellulaire. Toutefois, l'efficacité de ces thérapies est remise en cause, notamment de part la faible implantation cellulaire après injection et/ou les coûts très importants de ces traitements.

Il existe donc un réel besoin de trouver une nouvelle composition, traitement permettant de traiter les lésions tissulaires quelque soit leur origine et/ou d'augmenter l'efficacité de traitement des compositions connues et/ou des thérapies cellulaires tout en diminuant leur coût.

### Description de l'invention

La présente invention a précisément pour but de répondre à ces besoins en fournissant une composition pharmaceutique pour son application comme médicament pour la prévention et/ou le traitement de lésions tissulaires ladite composition comprenant
- un polymère biocompatible de formule générale (I) suivante

   AaXxYy (I)

   dans laquelle :
   A représente un monomère,
   X représente un groupement -R₁COOR₂ ou -R₉(C=O)R₁₀,
   Y représente un groupement O- ou N-sulfonate et répondant à l'une des formules suivante -R₃OSO₃R₄, -R₅NSO₃R₆, -R₇SO₃R₈
   dans lesquelles :
      R₁, R₃, R₅ et R₉ représentent indépendamment une chaîne hydrocarbonée aliphatique, éventuellement ramifiée et/ou insaturée et qui contient éventuellement un ou plusieurs cycles aromatiques, R₂, R₄, R₆ et R₈ représentent indépendamment un atome d'hydrogène ou un cation,
      R₇ et R₁₀ représente indépendamment une liaison, une chaîne hydrocarbonée aliphatique, éventuellement ramifiée et/ou insaturée,
      « a » représente le nombre de monomères,
      « x » représente le taux de substitution des monomères A par des groupements X,
      « y » représente le taux de substitution des monomères A par des groupements Y, et
- une cellule eucaryote.

Avantageusement, les inventeurs ont démontré de manière surprenante et contrairement aux enseignements de l'état de la technique que l'association de polymères biocompatibles de formule générale (I) tels que définis ci-dessus, également désignés RGTA dans la présente, et de cellules permet à la fois de préparer ou conditionner les tissus et organes, par exemple du patient receveur, de favoriser l'implantation, l'expansion et la colonisation des cellules externes et/ou de greffes d'organes et/ou de tissus, par exemple chez le patient receveur.

En particulier, les inventeurs ont démontré de manière surprenant que l'utilisation de polymères biocompatibles de formule générale (I) combiné avec des cellules, organes, extraits plaquettaires permet avantageusement et de manière surprenante d'améliorer notablement la récupération des fonctions altérées des tissus et/ou organes ainsi traités pour le plus grand bénéfice du patient.

En outre, les inventeurs ont démontré que la combinaison de polymères biocompatibles de formule générale (I) avec des cellules selon l'invention peut utilisée/appliquée pour un grand nombre de pathologie/lésions etc. En effet, la présente invention trouve une application quels que soient les tissus ou organes lésés/implantés ou greffés, quelles que soient les cellules utilisées, par exemple injectées, par exemple seules ou en combinaison par exemple avec des lysats plaquettaires par exemple enrichis en facteurs de croissance ou par exemple avec injection de facteurs de croissance purifiés, notamment des facteurs de croissances présentant une affinité pour les RGTA ou pour les héparanes sulfates.

Les inventeurs ont également démontrés de manière surprenante que l'effet obtenu sur la récupération fonctionnelle du tissu traité est supérieur à l'effet obtenu séparément soit par le RGTA seul ou par les cellules seules ou par le tissu ou l'organe ou le biomatériaux ou des lysats plaquettaires ou des facteurs de croissance purs implantés ou injectés seuls.

Les inventeurs ont également démontré de manière surprenante et inattendue que la posologie, schéma thérapeutique d'administration de la composition permet avantageusement d'optimaliser les effets thérapeutiques et en outre présente un fort effet synergique observé dans la récupération fonctionnelle et cicatricielle des tissus ou organes traités.

Les résultats observés sont d'autant plus inattendus que les cellules utilisées selon l'invention peuvent avoir des propriétés et des origines différentes de celles des cellules présentes dans les tissus ou organes dans lesquels elles sont susceptibles d'être implantées. D'une manière surprenante, la composition selon l'invention favorise la colonisation des cellules implantées au point, par exemple de reconstruire un tissu ou organe à nouveau fonctionnel malgré le fait que ces cellules implantées puissent être d'un phénotype et d'une origine tissulaire différente. En outre, sans que l'on ait besoin d'induire expérimentalement l'émergence de cellules souches pluripotentes ou déjà engagées dans la voie de différentiation des cellules du tissu ou organe à coloniser.

Les inventeurs ont démontré de manière surprenante que l'utilisation du RGTA selon l'invention permet d'obtenir un effet technique/thérapeutique nouveau et inattendu, à savoir notamment un conditionnement du site lésionnel par la préparation du terrain d'implantation et d'obtenir une implantation des cellules/ une efficacité du traitement thérapeutique bien supérieure à celui obtenu lors de l'utilisation du RGTA seul et/ou à celui des cellules utilisées seules, notamment concernant l'efficacité du traitement cellulaire et/ou la capacité d'implantation des cellules ou tissus ou organes.

Ainsi, les inventeurs ont démontré de manière surprenante et inattendue que la présente invention, via par exemple l'administration séquentielle, c'est-à-dire selon une posologie/régime posologique, de RGTA suivi après un certain délai allant, par exemple de quelques minutes à quelques jours, par l'administration de cellules de toutes natures et/ou d'origine choisies permet avantageusement de favoriser d'une manière inattendue la prise de ces greffes ou l'implantation fonctionnelle de ces cellules quelques en soit l'origine, leur stade de développement et le mode de préparation sous réserve de contrôler le cas échéant les aspects de rejet liés au système immunitaire du receveur.

En outre, les inventeurs ont démontré, notamment dans les exemples, que l'application de la présente invention, en particulier les lésions susceptibles d'être traitées par la composition selon l'invention peut être à tous types de lésions tissulaires quel qu'en soient l'origine ainsi qu'à tout type de tissu ou organe. En particulier, l'homme du métier, à la lumière des exemples ci-dessous dans lesquels une grande diversité de lésions sont effectivement traitées, comprend aisément et peut, à la lumière de ces connaissances extrapoler les autres lésions tissulaires susceptibles d'être traitées par la présente invention.

Dans la présente par « lesions tissulaires » on entend toute lésion de tout tissu biologique d'un mammifère connu de l'homme du métier. Il peut s'agir par exemple d'une lésion du tissu conjonctif, du tissu musculaire, du tissu nerveux, du tissu osseux, cartilagineux et/ou du tissu épithéliale. Il peut s'agir par exemple de toute lésion de tout organe, organelle de mammifère connu de l'homme du métier. Il peut s'agir par exemple d'une lésion de tissus du tractus digestif, de tissus du tractus gastro-intestinal, du système digestif alimentation et excrétion, du tractus génital, du système reproducteur, du système optique, olfactif ou auditif, du système sensoriel, du système circulatoire et/ou cardiovasculaire, du système respiratoire, du système musculaire, du système locomoteur. Il peut s'agir par exemple d'une lésion du tissu gastrique, d'une lésion buccale, d'une lésion de la cornée, d'une lésion tympanique, d'une lésion de la cochlée, d'une lésion de la peau, par exemple une plaie, une plaie chronique, par exemple une plaie du diabétique, une plaie ulcéreuse, un escarre, une brulure cutanée, une plaie nécrosante, une lésion veineuse, une lésion ischémique, par exemple une nécrose ischémique, une lésion due à un infarctus, par exemple une infarctus du myocarde, une lésion osseuse, par exemple une fracture, une fracture avec défaut osseux, une ostéonécrose (« non union bone fracture »), une lésion ostéochondrale, une lésion cartilagineuse, une lésion tendineuse, une lésion chirurgicale, une lésion due à une opération chirurgicale, une lésion due à un traitement médicale, par exemple une radiothérapie, une lésion du tissu nerveux, par exemple une lésion du cerveau, par exemple une lésion due à une exérèse d'une tumeur, une lésion de la moelle épinière, une lésion de fibre nerveuse, par exemple du système locomoteur et/ou sensoriel, une lésion du système respiratoire, par exemple des lésions pulmonaires, une lésion du système circulatoire, par exemple une lésion des artères et/ou des vaisseaux, une lésion du système digestif rénal, urinaire.

Dans la présente par monomère on entend par exemple un monomère choisi dans le groupe comprenant les sucres, les esters, les alcools, les acides aminés ou les nucléotides.

Dans la présente, les monomères A constituent les éléments de base des polymères de formule I peuvent être identiques ou différents.

Dans la présente, l'association de monomères peut permettre de former un squelette polymérique, par exemple un squelette polymérique de nature polyester, polyalcool, polysaccharidique, du type des acides nucléiques ou des protéines.

Dans la présente, parmi les polyesters, il peut s'agir par exemple de copolymères de biosynthèse ou synthèse chimique, par exemple des polyesters aliphatiques ou d'origine naturelle par exemple les polyhydroxyalconaotes.

Dans la présente, les polysaccharides et leurs dérivés peuvent être d'origine bactérienne, animale, fongique et/ou d'origine végétale. Il peut s'agir par exemple de polysaccharides à chaîne simple, par exemple les polyglucoses, par exemple le dextran, la cellulose, le bêta glucan, ou d'autres monomères comprenant des unités plus complexes, par exemple les xanthanes, par exemple le glucose, mannose et acide glucuronique ou encore des glucuronanes et glucoglucuronane.

Dans la présente, les polysaccharides d'origine végétale peuvent être à simple chaîne, par exemple la cellulose (glucose), les pectines (acide galacturonique), les fucanes, l'amidon ou plus complexe comme les alginates (acide galuronique et mannuronique),

Dans la présente, les polysaccharides d'origine fungique peuvent être par exemple le stéroglucane.

Dans la présente, les polysaccharides d'origine animale peuvent être par exemple les chitines ou le chitosan (glucosamine).

Le nombre de monomères A défini dans la formule (I) par « a » peut être tel que la masse desdits polymères de formule (I) est supérieure à environ 2000 daltons (ce qui correspond à 10 monomères de glucose). Le nombre de monomères A défini dans la formule (I) par « a » peut être tel que la masse desdits polymères de formule (I) est inférieure à environ 2000000 daltons (ce qui correspond à 10000 monomères de glucose). De façon avantageuse, la masse desdits polymères de formule (I) peut être comprise de 2 à 100 kdaltons.

Dans la présente, dans le groupement -R₁COOR₂ représentant X, R₁ peut être un alkyle en C₁ à C₆, par exemple un méthyl, un éthyl, un butyl, un propyl, un pentyl, de préférence un groupement méthyl, et R₂ peut être une liaison, un alkyle en C₁ à C₆, par exemple un méthyl, un éthyl, un butyl, un propyl, un pentyl, un groupement R₂₁R₂₂ dans lequel R₂₁ est un anion et R₂₂ un cation choisi dans le groupe des métaux alcalins.

De préférence, le groupement X est le groupement de formule -R₁COOR₂ dans laquelle R₁ est un groupement méthyle -CH₂- et R₂ un groupement R₂₁R₂₂ dans lequel R₂₁ est un anion et R₂₂ un cation choisi dans le groupe des métaux alcalins, de préférence le groupement X est un groupement de formule -CH₂-COO⁻.

Dans la présente, dans le groupement -R⁹(C=O)R₁₀ représentant X, R₉ peut être un alkyle en C₁ à C₆, par exemple un méthyl, un éthyl, un butyl, un propyl, un pentyl, de préférence un groupement méthyl, et R₁₀ peut être une liaison, un alkyle en C₁ à C₆, par exemple un méthyl, un éthyl, un butyl, un propyl, un pentyl, un hexyl.

Dans la présente, dans le groupement répondant à l'une des formules suivantes -R₃OSO₃R₄, -R₅NSO₃R₆, -R₇SO₃R₈ et représentant le groupement Y, R₃ peut être une liaison, un alkyle en C1 à C6, par exemple un méthyl, un éthyl, un butyl, un propyl, un pentyl, de préférence un groupement méthyl, R₅ peut être une liaison, un alkyle en C₁ à C₆, par exemple un méthyl, un éthyl, un butyl, un propyl, un pentyl, de préférence un groupement méthyl, R₇ peut être une liaison, un alkyle en C₁ à C₆, par exemple un méthyl, un éthyl, un butyl, un propyl, un pentyl, de préférence un groupement méthyl, R₄, R₆ et R₈ peuvent être indépendamment un atome d'hydrogène ou un cation M⁺, par exemple M⁺ peut être un métal alcalin.

De préférence, le groupement Y est le groupement de formule -R₇SO₃R₈ dans lequel R₇ est une liaison et R₈ est un métal alcalin choisi dans le groupe comprenant le lithium, le sodium, le potassium, le rubidium et le césium, De préférence, le groupement Y est un groupement -SO₃⁻Na⁺

Le taux de substitution de l'ensemble des monomères A par les groupements Y défini dans la formule générale (I) par « y » peut être compris de 30 à 150%, et de préférence de l'ordre de 100%.

Dans la présente, la définition des taux de substitutions ci- dessus, on entend par un taux de substitution « x » de 100%, le fait que chaque monomère A du polymère de l'invention contient statistiquement un groupement X. De même, on entend par un taux de substitution « y » de 100%, le fait que chaque monomère du polymère de l'invention contient statistiquement un groupement Y. Les taux de substitution supérieurs à 100% traduisent le fait que chaque monomère porte statistiquement plus d'un groupement du type considéré ; à l'inverse, les taux de substitution inférieurs à 100% traduisent le fait que chaque monomère porte statistiquement moins d'un groupement du type considéré.

Les polymères peuvent également comprendre des groupements chimiques fonctionnels, désignés Z, différents de X et Y.

Dans la présente, les groupements Z peuvent être identiques ou différent, et peuvent indépendamment être choisis dans le groupe comprenant des acides aminés, des acides gras, des alcools gras, des céramides, ou des dérivés de ceux-ci, ou des séquences nucléotidiques d'adressages.

Les groupements Z peuvent également représenter des agents actifs identiques ou différents. Il peut s'agir par exemple d'agents thérapeutiques, d'agents de diagnostic, d'un anti-inflammatoire, d'un antimicrobien, d'un antibiotique, d'un facteur de croissance, d'une enzyme.

Dans la présente, le groupement Z peut être avantageusement un acide gras saturé ou insaturé. Il peut s'agir par exemple d'un acide gras choisi dans le groupe comprenant l'acide acétique, l'acide caprylique, l'acide caprique, l'acide laurique, l'acide myristique, l'acide palmitique, l'acide stéarique, l'acide arachidique, l'acide béhénique, l'acide lignocérique, l'acide cérotique, l'acide myristoléique, l'acide palmitoléique, l'acide sapiénique, l'acide oléique, l'acide élaïdique, l'acide trans-vaccénique, l'acide linoléique, l'acide linolélaïdique, l'acide a-linolénique, l'acide γ-linolénique, l'acide dihomo-γ-linolénique, l'acide arachidonique, l'acide eicosapentaénoïque, l'acide clupanodonique ou l'acide docosahexaénoïque. De préférence, l'acide gras est l'acide acétique.

Dans la présente, le groupement Z peut être avantageusement un acide aminé de la série L ou D choisi dans le groupe comprenant l'alanine, l'asparagine, une chaine aromatique par exemple la tyrosine, la phénylalanine, le tryptophane, la thyroxine ou l'histidine.

Avantageusement, les groupements Z peuvent conférer aux polymères des propriétés biologiques ou physicochimiques supplémentaires. Par exemple les groupements Z peuvent augmenter la solubilité ou la lipophilie dudit polymère permettant par exemple une meilleure diffusion ou pénétration tissulaire.

Des polymères dans lesquels Z est présent répondent à la formule II suivante :

Aa Xx Yy Zz

dans laquelle, A, X, Y, a, x, y sont tel que défini ci-dessus et z représente le taux de substitution par des groupements Z.

Dans la présente le taux de substitution par des groupements Z représenté par « z » peut être compris de 0 à 50%, de préférence égale à 30%.

Les groupements X, Y et Z peuvent être indépendamment fixés sur le monomère A et/ou indépendamment fixés les uns aux autres. Lorsqu'au moins un des groupements X, Y et Z est indépendamment fixé sur un groupement X, Y et Z différent du premier, un desdits groupements X, Y ou Z est fixé au monomère A.

Ainsi, les groupements Z peuvent être fixés par covalence directement sur les monomères A ou fixés par covalence sur les groupements X et/ou Y.

Dans la présente, la composition peut comprendre une concentration de 0,01 microgramme à 100 mg en poids de polymère biocompatible par rapport au poids total de la composition. Par exemple la composition peut comprendre de 10 microgrammes à 10 milligrammes en poids par rapport au poids total de la composition.

Dans la présente, la composition peut être formulée et/ou adaptée selon son administration. Par exemple, pour une administration par voie intraveineuse ou par voie intramusculaire la composition peut être administrée afin de délivrer une dose de polymère biocompatible comprise de 0,1 à 5 mg par kilogramme de poids corporel, ou pour une administration par voie orale la composition peut être administrée, par exemple en 2 à 5 prises égales par jour à hauteur d'un total quotidien par exemple de 1 à 500 mg, par exemple 10 microg à 5 mg par kg de polymère biocompatible, par exemple de 10 microg à 5 mg par kg, par exemple pour un adulte de 100 kg de masse corporelle pendant plusieurs jours à plusieurs semaines. Par exemple, pour une administration par voie intracrânienne, il peut s'agir d'une administration unique de 1 à 5 mL, ou par minipompe sur plusieurs jours, la composition peut comprendre une concentration de 0,001 à 1 mg.mL⁻¹ de polymère biocompatible, ou pour une administration, par exemple quotidienne de 1 à 5mL, par voie sublinguale la composition peut comprendre une concentration de 0,1 à 100 mg.mL⁻¹ de polymère biocompatible, ou par voie aérienne la composition peut être administrée afin de délivrer une dose comprise de 0,1 à 5 mg de polymère biocompatible par kilogramme de poids corporel dudit polymère.

Par exemple, pour une administration par voie orale, le polymère peut être en solution, par exemple dans de l'eau ou dans tout solvant adapté pour une administration par voie orale connue de l'homme du métier. La composition peut être également en cachet, gélule ou toute autre forme compatible avec une prise orale connue de l'homme du métier. Il peut s'agir d'une solution aqueuse d'un volume de 10 à 50 mL comprenant par exemple une concentration de 0.1mg.mL⁻¹ de polymère dans la solution. Par exemple, pour une administration par voie systémique, le polymère peut être par exemple en solution dans du sérum physiologique, par exemple du sérum physiologique de qualité injectable ou toute autre forme compatible avec l'injection, par exemple des solutions avec du glucose et/ou d'autres excipients adaptés connus de l'homme du métier, par exemple comprenant des polysaccharides, par exemple l'héparine. Par exemple, le polymère peut être à une concentration de 0,1 à 5 mg.kg⁻¹, de préférences de 1 à 2,5 mg.kg⁻¹, par exemple pour administration par voie Intra-Veineuse (IV) et/ou Intra-Musculaire(IM).

Selon l'invention, la composition peut comprendre en outre des facteurs de croissance. Il peut s'agir de tout facteur de croissance connu de l'homme du métier susceptible de favoriser, stimuler la croissance cellulaire. Il peut s'agir par exemple de facteurs de croissance choisis dans le groupe comprenant le facteur de croissance des fibroblastes (FGF), par exemple le FGF1 ou FGF2, le facteur de croissance vasculaire endothéliale (VEGF), le facteur de croissance dérivé des plaquettes (PGDF) ou un mélange de ceux-ci. Selon l'invention, la composition peut comprendre de 10 nanog.mL⁻¹ à 100 microg.mL⁻¹ de facteurs de croissance.

Selon l'invention, la composition peut comprendre en outre un extrait ou lysat plaquettaire brut ou enrichi en facteurs de croissance. Il peut s'agir par exemple de tout extrait ou lysat plaquettaire connu de l'homme du métier et/ou disponible dans le commerce.

Dans la présente par « cellule eucaryote » on entend toute cellule eucaryote connue de l'Homme du métier. Il peut s'agir par exemple d'une cellule eucaryote de mammifère, par exemple une cellule eucaryote animale ou humaine. Il peut s'agir par exemple de toute cellule eucaryote quelque soit son stade de différenciation, par exemple une cellule choisie dans le groupe comprenant les cellules eucaryotes adultes ou embryonnaires, les cellules souches embryonnaires, les cellules souches adultes. Il peut s'agir par exemple de cellules eucaryotes de sang de cordon, de cellules de la moelle osseuse, de cellules du tissu adipeux, de cellules mésenchymateuses. Il peut s'agir également d'un groupe de cellules, d'un greffon et/ou d'un organe.

Il peut s'agir par exemple d'une cellule hétérologue, homologue ou autologue d'un individu. De préférence, les cellules sont des cellules autologues.

Il peut s'agir également d'une cellule souche pluri ou totipotente, ou de cellules engagées dans des voies de différenciations, par exemple des cellules souches mésenchymateuses. Il peut s'agir également d'une cellule souche pluri ou totipotente à l'exception des cellules souches embryonnaires.

Avantageusement, lorsque les cellules sont autologues, la composition selon l'invention la présente invention peut être préférée pour des raisons réglementaires, de sécurité, de faisabilité, d'efficacité et économique.

Avantageusement, lorsque les cellules sont autologues, elles sont de préférence isolées de l'individu et utilisées dans la composition selon l'invention et/ou utilisées dans un traitement dans les 24 heures après prélèvement et isolement sans autres ajouts. Avantageusement, cette administration unique permet de surmonter et de ce conformer aux exigences/contraintes réglementaires.

Dans la présente la quantité de cellules comprises dans la composition peut être de 1 à 5x10⁷ cellules.

Avantageusement, les inventeurs ont démontré que le traitement est indépendant du type cellulaire, de son origine, des moyens utilisés pour sélectionner, amplifier, conditionner et modifier les cellules. En particulier, les inventeurs ont démontré de manière surprenante que la présente invention s'applique donc à toutes cellules quelques soient leurs provenances et les manipulations de sélections conduisant à engager les cellules dans des voies de différentiation ou de dédifférenciation dans la mesure où l'administration du RGTA permet avantageusement, notamment lors de son association avec l'administration de cellules, la création/formation d'un microenvironnement adapté au cellule permettant avantageusement un coopération synergique entre le RGTA et les cellules permettant d'augmenter l'implantation cellulaire.

Selon l'invention, lorsque la composition comprend un greffon ou un organe pour greffe, ou un matériau implantable l'administration du polymère et du greffon ou organe ou du matériau implantable peut être simultanée. Par exemple l'organe à greffer ou le matériau implantable peut être imprégné, par exemple soit en le plongeant dans une solution comprenant le biopolymère, soit par perfusion, soit par spray ou tout autre méthode adaptée connue de l'homme du métier. Dans le cas de matériau implantable, le polymère peut être incorporé dès la fabrication du matériau implantable permettant avantageusement une préincubation ou imprégnation.

Selon l'invention, la durée d'imprégnation peut être comprise de quelques minutes à plusieurs heures, par exemple de 1 minutes à 18 heures, de 5 minutes à 16 heures une nuit.

Avantageusement, lorsque le greffon ou organe est imprégnée pendant une durée supérieure à 5 minutes, par exemple de 16 heures l'imprégnation permet en outre d'améliorer l'efficacité de prise de greffe. En effet, les inventeurs ont montré de manière surprenante que la présence du polymère dans la solution de conservation du greffon et/ou de l'organe présente avantageusement un effet protecteur et anti apoptotique.

Avantageusement, le greffon, l'organe ou le matériau imprégné du polymère peut recevoir avant ou après la réimplantation ou la greffe une administration de facteurs de croissance ou d'extrait plaquettaire afin de favoriser la colonisation par les cellules administrées au moment de l'implantation ou après.

Dans la présente, par « composition pharmaceutique » on entend toute forme de composition pharmaceutique connue de l'Homme du métier. Dans la présente, la composition pharmaceutique peut être par exemple une solution injectable, par exemple pour une injection locale ou systémique, par exemple en sérum physiologique, en solution glucose injectable, en présence d'excipients, par exemple de Dextrans, par exemple a des concentrations connues de l'homme du métier, par exemple du microgramme à quelques milligrammes par mL.

La composition pharmaceutique peut être par exemple un médicament destiné à une administration orale choisie dans le groupe comprenant une formulation liquide, une forme posologique effervescente orale, une poudre orale, un système multiparticule, une forme galénique orodispersible.

Par exemple, lorsque la composition pharmaceutique est pour administration orale, elle peut être sous la forme d'une formulation liquide choisie dans le groupe comprenant une solution, un sirop, une suspension, une émulsion. Lorsque la composition pharmaceutique est sous la forme d'une forme posologique effervescente orale, elle peut être sous une forme choisie dans le groupe comprenant les comprimés, les granules, les poudres. Lorsque la composition pharmaceutique est sous la forme d'une poudre orale ou un système multiparticulaire, il peut être sous une forme choisie dans le groupe comprenant des billes, des granulés, des mini-comprimés et les microgranules. Lorsque la composition pharmaceutique est sous la forme d'une forme posologique orodispersible, elle peut être sous une forme choisie dans le groupe comprenant des comprimés orodispersibles, gaufrettes lyophilisées, films minces, un comprimé à mâcher, d'un comprimé, d'une capsule ou d'une gomme médicale à mâcher.

Selon la présente invention, la composition pharmaceutique peut être une composition pharmaceutique pour administration par voie orale, par exemple buccale et/ou sublingual, par exemple choisie dans le groupe comprenant les comprimés buccaux ou sublinguaux, les pastilles, gouttes, une solution pour pulvérisations.

Selon la présente invention, la composition pharmaceutique peut être une composition pharmaceutique pour administration topique, transdermique, par exemple choisie dans le groupe comprenant les pommades, crèmes, gels, lotions, patchs et mousses.

Selon la présente invention, la composition pharmaceutique peut être une composition pharmaceutique pour administration nasale, par exemple choisie dans le groupe comprenant des gouttes nasales, spray nasal, de la poudre nasale.

Selon la présente invention, la composition pharmaceutique peut être une composition pharmaceutique pour administration parentérale, par exemple sous-cutanée, intramusculaire, intraveineuse, intrathécale.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes qui peuvent se développer chez un patient présentant une lésion tissulaire.

Dans la présente, l'administration du polymère biocompatible et de la cellule peut être simultanée, successive ou concomitante.

Selon l'invention, au moins une des administrations peut être réalisée par voie orale ou par injection. Les deux administrations peuvent être réalisées de la même manière ou différemment. Par exemple, l'administration du polymère biocompatible et des cellules peut être faite par injection, l'administration du polymère biocompatible peut être par voie orale et les cellules peut être faite par injection locale. L'administration peut être également fonction du site de la lésion.

Selon l'invention, l'utilisation de cellule eucaryote, notamment leur administration peut être réalisée dans un délai de 5 minutes à 1 une semaine après la première administration dudit polymère biocompatible.

Dans le cas de greffe de tissus, d'organes ou de l'utilisation de matériaux implantables pré-imprégnés par le polymère, l'administration de cellule peut se faire avant l'implantation ou juste après, par exemple dans l'heure.

Selon l'invention, la composition peut être, par exemple, administrée quotidiennement, biquotidiennement et hebdomadairement. Il peut s'agir par exemple d'une administration une fois par jour, deux fois par jour ou plus.

Selon l'invention, la composition peut être, par exemple, administrée sur une durée de 1 jour à 3 mois, par exemple pendant 2 mois. Par exemple, la composition peut être administrée sur une période de 3 mois avec une fréquence d'administration tous les 15 jours.

Selon l'invention, le biopolymère peut être, par exemple, administré sur une durée de 1 jour à 3 mois, par exemple pendant 2 mois, avec par exemple une fréquence de 1 fois par jour et la cellule eucaryote administrée sur une durée identique ou différente, par exemple une durée de 1 jour à 3 mois, avec une fréquence hebdomadaire.

Selon l'invention, lorsque l'administration des polymères et des cellules sont successives, la posologie pour chaque administration peut être une administration des polymères suivie de l'administration des cellules. Par exemple, les cellules peuvent être administrées de 1 minute à 24 heures après l'administration des polymères, de 30 minutes à 12 heures après administration des polymères, de 45 minutes à 6 heures après administration des polymères, 1 heure après administration des polymères.

Selon l'invention, la composition peut comprendre en outre un extrait plaquettaire. Il peut s'agir par exemple de tout extrait plaquettaire connu de l'homme du métier adapté pour être administré à un mammifère. Il peut s'agir par exemple d'un extrait plaquettaire disponible dans le commerce, d'un extrait plaquettaire obtenu selon le procédé décrit dans le document D R Knighton, et al., Ann Surg. Sep 1986; 204(3): 322-330 « Classification and treatment of chronic nonhealing wounds. Successful treatment with autologous platelet-derived wound healing factors (PDWHF) » ou dans les documents Everts, P.A.M et al., Autologous platelet gel growth factor release and leukocyte kinetics using three devices. Transf. Med., 2006, 16(5), 363-368 ou « Is Use of Autologous Platelet-Rich Plasma Gels in Gynecologic, Cardiac, and General, Reconstructive Surgery Beneficial ?" Pharmaceutical Biotechnology, 2012, Vol. 13N°13). Il peut s'agir d'extrait plaquettaire correspondant à des lysats plaquettaires enrichis en activité facteur de croissance administrable, par exemple par voie locale, injectable ou déposés dans des supports variés, par exemple dans ou sur des gels, crèmes, sprays et/ou dans des tissus lésés. Selon l'invention, l'extrait plaquettaire peut être un extrait autologue ou hétérologue d'un individu à traiter. De préférence, l'extrait plaquettaire est un extrait autologue. Il peut s'agir par exemple d'un extrait et/ou lysat plaquettaire concentré, par exemple par des procédés et/ou kits connu de l'homme du métier et/ou disponible dans le commerce.

Selon l'invention, la composition peut comprendre de 0,1mL à 100mL d'extrait et/ou de lysat plaquettaire.

Avantageusement, l'extrait plaquettaire comprend des facteurs de croissance favorisant la formation d'un « environnement » propice à l'implantation de cellules. Ces facteurs peuvent être ajoutés à l'extrait plaquettaire ou utilisés seuls sans extraits. Selon l'invention le facteur FGF1 ou FGF2 ou VEGF ou TGFbeta1 ou BMP ou d'autres facteurs de croissances, de préférence au moins un facteur choisi dans le groupe comprenant le facteur FGF1 ou FGF2 ou VEGF ou TGFbeta1 ou BMP peuvent être ajoutés. Selon l'invention, les facteurs compris dans l'extrait plaquettaire peut être ajouté à des concentrations de 10 ng à 100 microg/ml d'extrait.

Selon l'invention, préalablement à l'administration de la composition, le tissu lésé peut être « réactivé » par exemple par réouverture de la lésion, par grattage ou par détersion mécanique au scalpel. La réactivation peut être mise en oeuvre, par exemple, sur des plaies anciennes, par exemple des fractures osseuses consolidées, des vieilles cicatrices ou des plaies chroniques.

La présente invention a également pour objet une composition pharmaceutique ou dermatologique pour son application comme médicament pour la prévention et/ou le traitement de lésions tissulaires, ladite composition comprenant un biopolymère de formule AaXxYy ou AaXxYyZz et au moins un extrait plaquettaire.

Selon l'invention, l'extrait plaquettaire est tel que défini ci-dessus.

Selon l'invention, le biopolymère de formule AaXxYy ou AaXxYyZz est tel que défini ci-dessus.

Selon l'invention, la composition pharmaceutique ou dermatologique est telle que définie ci-dessus.

Selon l'invention la fréquence d'administration du polymère biocompatible peut être telle que définie ci-dessus.

Selon l'invention, le mode et/ou la voie d'administration du polymère biocompatible peut être tel(s) que défini(s) ci-dessus.

La présente invention a également pour objet une composition pharmaceutique ou dermatologique pour son application comme médicament pour la prévention et/ou le traitement de lésions tissulaires, ladite composition comprenant un biopolymère de formule AaXxYy ou AaXxYyZz et au moins un facteur de croissance.

Selon l'invention, le facteur de croissance est tel que défini ci-dessus. Selon l'invention, le biopolymère de formule AaXxYy ou AaXxYyZz est tel que défini ci-dessus.

La composition pharmaceutique ou dermatologique est telle que définie ci-dessus.

Selon l'invention la fréquence d'administration du polymère biocompatible peut être telle que définie ci-dessus.

Selon l'invention, le mode et/ou la voie d'administration du polymère biocompatible peut être tel(s) que défini(s) ci-dessus.

La présente invention se rapporte également à un procédé de traitement d'un patient ayant subi lésion tissulaire comprenant dans un ordre quelconque les étapes suivantes:
i.l'administration d'au moins un polymère biocompatible, et
ii. l'administration d'au moins une cellule eucaryote, dans lequel, les administrations sont concomitantes, successives ou alternatives.

Le polymère biocompatible est tel que défini ci-dessus.

La cellule eucaryote est telle que définie ci-dessus.

Selon l'invention, le patient peut être tout mammifère. Il peut s'agir par exemple d'un animal ou d'un être humain.

Selon l'invention, la cellule eucaryote administrée peut être une cellule hétérologue ou homologue du dit patient.

Selon l'invention, le mode et/ou la voie d'administration du polymère biocompatible peut être tel(s) que défini(s) ci-dessus.

Selon l'invention, le mode et/ou la voie d'administration de la cellule peut être tel(s) que défini(s) ci-dessus.

Selon l'invention la fréquence d'administration du polymère biocompatible peut être telle que définie ci-dessus.

Selon l'invention la fréquence d'administration de la cellule eucaryote peut être telle que définie ci-dessus.

Selon l'invention, lorsque l'administration des polymères biocompatibles et des cellules sont successives, la posologie pour chaque administration peut être une administration des polymères biocompatibles suivie de l'administration des cellules. Par exemple, les cellules peuvent être administrées de 1 minute à 48 heures après l'administration des polymères biocompatibles, de 30 minutes à 12 heures après administration des polymères, de 45 minutes à 6 heures après administration des polymères, 1 heure après administration des polymères.

Avantageusement, la cellule eucaryote est une cellule souche adulte mésenchymateuse.

La présente invention se rapporte également à un procédé de traitement d'un patient ayant subi lésion tissulaire comprenant dans un ordre quelconque les étapes suivantes:
i.l'administration d'au moins un polymère biocompatible, et
ii. l'administration d'au moins un extrait et/ou lysat plaquettaire, dans lequel, les administrations sont concomitantes, successives ou alternatives.

Le polymère biocompatible est tel que défini ci-dessus.

L'extrait et/ou lysat plaquettaire est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention la fréquence d'administration du polymère biocompatible peut être telle que définie ci-dessus.

Selon l'invention, lorsque l'administration des polymères biocompatibles et de l'extrait et/ou lysat plaquettaire sont successives, la posologie pour chaque administration peut être une administration des polymères biocompatibles suivie de l'administration de l'extrait et/ou lysat plaquettaire. Par exemple, l'extrait et/ou lysat plaquettaire peut être administré immédiatement, c'est-à-dire de manière concomitante, ou quelques minutes, de préférence de 10 minutes à quelques heures, par exemple de 1 minutes à 120 minutes, de préférence de 10 à 60 minutes après l'administration, par exemple, locale ou une injection des polymères biocompatibles, après plusieurs heures, de préférence de 4h à 24h, par exemple après administration du polymère biocompatible par voie orale.

La présente invention se rapporte également à un procédé de traitement d'un patient ayant subi lésion tissulaire comprenant dans un ordre quelconque les étapes suivantes:
i.l'administration d'au moins un polymère biocompatible, et
ii. l'administration d'au moins un facteur de croissance,
   dans lequel, les administrations sont concomitantes, successives ou alternatives.

Le polymère biocompatible est tel que défini ci-dessus.

Le facteur de croissance est tel que défini ci-dessus.

Selon l'invention, le patient peut être tout mammifère. Il peut s'agir par exemple d'un animal ou d'un être humain.

Selon l'invention, le mode et/ou la voie d'administration du polymère biocompatible peut être tel(s) que défini(s) ci-dessus.

Selon l'invention la fréquence d'administration du polymère biocompatible peut être telle que définie ci-dessus.

Selon l'invention, lorsque l'administration des polymères biocompatibles et dudit au moins un facteur de croissance sont successives, la posologie pour chaque administration peut être une administration des polymères biocompatibles suivie de l'administration dudit au moins un facteur de croissance. Par exemple, ledit au moins un facteur de croissance peut être administré immédiatement, c'est-à-dire de manière concomitante, ou par exemple de 1 minutes à quelques heures, par exemple de 1 minutes à 120 minutes, de préférence de 10 à 60 minutes après l'administration par exemple, locale ou une injection des polymères biocompatibles, après plusieurs heures, de préférence de 4h à 24h, après administration du polymère biocompatible par voie orale.

En d'autres termes, même si dans la présente description il est fait référence à une composition, on comprend bien que chacun des composés de la composition peut être administré concomitamment aux autres composés (par exemple dans une seule composition ou dans deux compositions, chacune de ces compositions comprenant un ou plusieurs des composants précités, le mode d'administration de chacun des composés ou composition(s) pouvant être identique ou différent), ou indépendamment les un des autres, par exemple successivement, par exemple administration indépendante d'un polymère biocompatible et, administration indépendante d'une cellule eucaryote, ces administrations étant réalisées sur un même patient, concomitamment ou successivement ou alternativement, dans un ordre qui est celui précité ou un autre ordre. Ces différentes administrations peuvent être réalisées, indépendamment l'une de l'autre ou de manière liée (composition ou co-administration), par un mode d'administration identique ou différent (injection, ingestion, application topique, etc.), une ou plusieurs fois par semaine, pendant un ou plusieurs semaines successifs ou non.

La présente invention a également pour objet un kit pharmaceutique pour la prévention et/ou le traitement de lésions tissulaires comprenant :
i.un polymère biocompatible, et
ii. au moins une cellule eucaryote.

Le polymère biocompatible est tel que défini ci-dessus.

La cellule eucaryote est telle que définie ci-dessus.

La présente invention a également pour objet un kit pharmaceutique pour la prévention et/ou le traitement de lésions tissulaires comprenant :
i.un polymère biocompatible, et
ii. au moins un extrait et/ou lysat plaquettaire.

Le polymère biocompatible est tel que défini ci-dessus.

L'extrait et/ou lysat est tel que défini ci-dessus.

La présente invention a également pour objet l'utilisation d'une composition pharmaceutique comprenant
i.un polymère biocompatible, et
ii. au moins un facteur de croissance
pour la fabrication d'un médicament pour le traitement de lésions tissulaires.

Le polymère biocompatible est tel que défini ci-dessus.

Le facteur de croissance est tel que défini ci-dessus.

Dans ce mode de réalisation, par médicament on entend une composition pharmaceutique telle que définie ci-dessus.

Avantageusement, les inventeurs ont démontré que l'administration séquentielle de RGTA suivi après un certain délai allant de quelques minutes à quelques jours de cellules, quelque soit leurs natures et origines permet avantageusement et de manière inattendue de favoriser l'implantation fonctionnelle desdites cellules ou la prise de greffes. En outre, les inventeurs ont également démontré que les avantages précités ont été également observés quelques en soit l'origine, le stade de développement et/ou le mode de préparation des cellules utilisées.

Avantageusement, l'invention fournie donc une réponse générale et simple à un problème technique complexe et pour lequel un besoin réel et persistant existe dans l'état de la technique. Elle est notamment illustrée de manière non limitative dans les exemples ci-dessous facilement extrapolable pour l'homme de l'art à tous types de lésions tissulaires quel qu'en soient l'origine ainsi qu'à tout type de tissu ou organe.

En particulier, les inventeurs ont démontré que la présente invention peut être généralisée à l'ensemble des lésions tissulaires notamment de part certaines caractéristiques communes des lésions tissulaires comprenant une destruction de cellules et de la matrice extracellulaire au niveau du site lésionnel.

La présente invention a également pour objet un procédé ex-vivo de préparation de greffe comprenant l'imprégnation d'un greffon et/ou organe à greffer dans une solution comprenant un polymère biocompatible.

La présente invention a également pour l'utilisation du biopolymère de formule AaXxYy ou AaXxYyZz tel que défini ci-dessus pour la préparation ex-vivo d'un greffon et/ou organe.

Le polymère biocompatible est tel que défini ci-dessus.

Selon l'invention, l'imprégnation peut être réalisée par tout procédé connu de l'homme du métier. Il peut s'agir par exemple de plonger l'organe et/ou le plongeant dans une solution comprenant le biopolymère, soit par perfusion, soit par spray.

La présente invention a également pour objet l'utilisation du biopolymère de formule AaXxYy ou AaXxYyZz tel que défini ci-dessus pour la préparation in-vitro et/ou ex-vivo d'un biomatériau implantable.

Par exemple pour des biomatériaux implantables, le polymère biocompatible peut être ajouté par exemple par imprégnation après fabrication du biomatériau, par exemple pour un tissu ou un organe. Il peut être par exemple aussi ajouté lors de la fabrication du biomatériau dès le départ, par exemple le biopolymère de formule AaXxYy ou AaXxYyZz tel que défini ci-dessous peut être ajouté par couches successives par exemple de manière similaire a des impressions 3D

Dans la présente, par « biomatériaux implantables » ont entend tout biomatériaux implantables connus de l'homme du métier et/ou disponible dans le commerce. Il peut s'agir par exemple d'un matériau implantable compatible, par exemple de toute nature, biodégradable, réticulés ou non, colonisable de préférence. Il peut s'agir de biomatériaux implantables à base de réticulation de protéines, par exemple de collagènes, de fibrine, de polysaccharides par exemple le dextran, la chitine, l'acide hyaluronique, l'alginate, la cellulose et leurs dérivatifs, de copolymères biodégradables et biocompatibles à base d'acide glycolique, lactique, malique, de polymères, par exemple pouvant prendre des transitions liquides gel par des polymérisation contrôlable par la température, ou par des enzymes ou des irradiations ou autres procédés. Il peut s'agir par exemple de polymère à base de polycaprolactone, polyurétane, polytétrafluoroethylène silicone, à base de sels inorganiques par exemple les phosphates de calcium ou les hydroxyapatite. Il peut s'agir par exemple de matériaux à base ou en céramique, en métal, par exemple en aluminium, en acier, en titalen et/ou des alliages de ceux-ci. Avantageusement lorsque le matériau est un matériau à base ou en céramique et/ou en métal, l'imprégnation permet recouvrement de la surface extérieure du matériau, l'imprégnation peut être avantageusement réalisée par spray.

Selon l'invention, la solution d'imprégnation peut comprendre une concentration 0,1microg.mL⁻¹ à 1mg.mL⁻¹ de polymère biocompatible tel que défini ci-dessus.

Selon l'invention, la durée d'imprégnation peut être comprise de 5 minutes à 24 heures. Avantageusement, la durée d'imprégnation peut être fonction de la structure du greffon et/ou organe et/ou du matériau implantables.

Avantageusement, l'imprégnation permet en outre d'améliorer l'efficacité de prise de greffe. En effet, les inventeurs ont montré de manière surprenante que la présence du polymère dans la solution de conservation du greffon et/ou de l'organe présente avantageusement un effet protecteur et anti apoptotique.

L'imprégnation peut être avantageusement complétée, après l'imprégnation par le polymère biocompatible par une imprégnation de facteurs de croissance et/ou d'extraits plaquettaires tel que mentionné ci-dessus. Selon l'invention, la durée d'imprégnation de facteurs de croissance et/ou d'extraits plaquettaires peut être comprise de 5 minutes à 24 heures.

L'imprégnation peut être avantageusement complétée, par imprégnation et/ou déposition ex-vivo et/ou in-vitro de cellules sur le biomatériau implantable imprégné par le polymère biocompatible.

Selon l'invention les cellules sont tels que définies ci-dessus.

Selon l'invention, la solution d'imprégnation et/ou de déposition de cellules peut comprendre une concentration quelques milliers a quelques millions de cellules, de préférence de 10 000 à 1 000 000 de cellules.

Selon l'invention, la durée d'imprégnation et/ou de déposition ex-vivo et/ou in-vitro de cellules peut être comprise de 5 minutes à 24 heures.

Dans la présente, l'imprégnation et/ou la déposition de cellules sur le biomatériau implantable imprégné par le polymère biocompatible peut être réalisé par tout procédé adapté connu de l'homme du métier.

En outre, selon l'invention, l'imprégnation biomatériaux implantable peut être avantageusement complétée, après l'imprégnation par le polymère biocompatible et/ou imprégnation ou déposition ex-vivo et/ou in-vitro de cellules, par une imprégnation d'au moins un facteur de croissance.

Selon l'invention, l'imprégnation sur le biomatériaux implantable imprégné par au moins un facteur de croissance peut être réalisée par tout procédé adapté connu de l'homme du métier.

Selon l'invention le facteur de croissance est tel que défini ci-dessus.

Selon l'invention, la solution d'imprégnation d'au moins un facteur de croissance peut comprendre une concentration de 10 ng/ml à 100 microg/ml de facteurs de croissance.

Selon l'invention l'extrait plaquettaire et/ou lysat plaquettaire est tel que défini ci-dessus. Il peut s'agir par exemple d'un extrait plaquettaire concentré, par exemple à partir de 5 à 100ml de sang via, par exemple, l'utilisation de kits de concentration plaquettaire connu de l'homme du métier et disponibles dans le commerce, par exemple les Kit de Curasan (marque déposée), Plateltex (marque déposée), GPS (marque déposée) Il et RegenLab (marque déposée).

Selon l'invention, la solution d'imprégnation peut comprendre une concentration de 0,1.à 6ml d'extrait plaquettaire.

D'autres avantages pourront encore apparaître à l'Homme du métier à la lecture des exemples ci-dessous, illustrés par les figures annexées, donnés à titre illustratif.

### Exemples

### Exemple 1: Préparation des RGTA et mode d'administration:

La synthèse des RGTA est largement décrite dans l'art antérieur, par exemple dans le brevet intitulé- « PROCEDE DE SULFONATION DE COMPOSES COMPRENANT DES GROUPEMENTS HYDROXYLES (OH) LIBRES OU DES AMINES PRIMAIRES OU SECONDAIRES » et également dans la référence bibliographique : Yasunori I. et al., Biomaterials 2011, 32 :769e776) et Petit E. et al,. Biomacromolecules. 2004 Mar-Apr; 5(2):445-52.

Dans les exemples ci-dessous, plusieurs RGTA connus et décrits ont été utilisés dont le OTR4120 (décrit dans Khammari-Chebbi et al. , J Fr Ophtalmol. 2008 May;31(5):465-71) et le OTR4131 (décrit dans Frescaline G. et al., Tissue Eng Part A. 2013 Jul;19(13-14):1641-53. doi: 10.1089/ten.TEA.2012.0377) disponibles dans le commerce. En outre, le composé OTR4131 est un composé comprenant un radical Z qui est un acide gras, à savoir l'acide acétique décrit dans Virginie Coudry, et al. Long-Term Follow-up of Superficial Digital Flexor TendonitisTreated by a Single Intralesional Injection of a ReGeneraTing Agent in 51 Horses Journal of Equine Veterinary Science 34 (2014) 1357-1360 et un composé dans lequel Z est un acide aminé comme la phénylalanine décrit dans les brevets américains US 7,998,922 et US patent 8,790,631 ont été également utilisé dans les exemples ci-dessous.

Dans les exemples ci-dessous, l'administration a été réalisée comme décrit précédemment. En d'autres termes lorsque le RGTA n'est pas mélangé dans une solution unique avec les cellules, celui-ci a été administré selon les modes d'administration connus pour ce composé.

Avantageusement, lorsque le RGTA est administré via une composition indépendante un des effets obtenu peut être une préparation du tissu ou de l'organe à traiter pour favoriser une implantation, colonisation et expansion par des cellules choisies permettant une réparation et régénération tissulaire plus efficace et synergique de part la combinaison RGTA et cellules.

La fréquence d'administration peut être unique ou réitérée toutes les semaines ou quinze jours voir mensuelles, rarement pour une durée totale de plusieurs mois, dépendant du succès de la colonisation et du nombre d'injection/administration nécessaire de cellules, le temps nécessaire pour la préparation de la niche avant l'implantation.

Les concentrations et doses du RGTA administrées dépendaient des formes d'administrations locales ou systémiques, des fréquences, des tissus, organes ou zones à traiter, volumes ou surface de la lésion.

Dans les exemples ci-dessous lorsque l'administration est effectuée par voie orale, les RGTA peuvent être en solution dans de l'eau ou sous forme de toute autre présentation buvable mais également en cachet, gélule ou toute autre forme compatible avec une prise orale. En outre, avantageusement, les RGTA administrés par voie orale présentes une résistance à la dégradation par l'acide et par les sucs digestifs remarquable. Avantageusement, le produit n'ayant aucun gout et parfaitement soluble dans l'eau, la prise préférée est sous forme d'une solution aqueuse dans un volume de 10 à 25 mL et à une concentration de 0,1mg.mL⁻¹ afin que la quantité soit de 1 à 2,5 mg par prise, deux à cinq fois par jour. Dans la majorité des exemples, ces prises étaient matin à jeun et le soir au coucher pour des poids des patients variant de 50 à 100 kg, les doses et fréquences pouvant elle aussi varier, ainsi globalement la prise quotidienne a été entre 1 et 500mg/jour. La durée de cette administration peut être de plusieurs mois sans que d'effets adverses aient été observées. En particulier, une prise par un individu de 25 mg/jour sur plus d'une année n'a induit aucune gêne ni effets secondaires apparents.

Dans le cas de traitement du tractus digestif avec des cellules afin de réparer et régénérer des lésions, les quantités de RGTA peuvent être avantageusement réduites. En effet, les molécules de RGTA peuvent avantageusement rentrer directement avec le tissu lésé et, par exemple recréer une niche, permettant avantageusement une meilleure implantation des cellules et le cas échéant de favoriser leur prolifération. Les doses utilisées lors de traitement du tractus digestif peuvent être, par exemple de 1 à 50 mL à 100 microg.mL⁻¹ par jour. Il peut s'agir par exemple de doses identiques ou similaires à celle décrites dans le document Meddahi et al., J Biomed Mater Res 60: 497-501 2002, par exemple pour le traitement des ulcères gastriques ou digestifs, de doses identiques ou similaires à celle décrites dans le document Alexakis et al., Gut 2004;53:85-90 pour le traitement de pathologies de Crohn, ou de doses identiques ou similaires à celle décrites dans le document Alexakis et al., FASEB J. 2001, 15,1546-1554 pour des tissus lésés après irradiation.

Dans les exemples ci-dessous lorsque l'administration est effectuée par injection systémique, les RGTA étaient de préférence en solution dans du sérum physiologique de qualité injectable ou toute autre forme compatible avec l'injection, notamment des solutions avec du glucose ou d'autres excipients usuels avec des polysaccharides comme l'héparine, ou mélangés avec des produits thérapeutiques ayant d'autres propriété sous réserve d'avoir évalué le risque d'interactions avec d'autres principes actifs. Les RGTA ont été utilisés à des concentrations de 0,1 à 5 mg.kg⁻¹, de préférences de 1 à 2,5 mg.mL⁻¹ par voie Intra-Veineuse (IV) ou Intra-Musculaire (IM). Pour cette voie d'injection, il peut s'agir d'une injection unique, quotidienne ou hebdomadaire.

Dans les exemples ci-dessous lorsque cela a été possible l'administration en locale dans ou à proximité du tissu ou du site lésé a été privilégié. En particulier, cette voie d'administration a été privilégiée pour les muqueuses, par exemple buccales, vaginales, urétrales, digestives avec accès endoscopique ou vasculaire ou cardiaque, et/ou dans les zones d'accès plus difficiles mais susceptible d'être directement atteignables comme la moelle épinière, la zone péri rétinienne, intra-ventriculaire, les voies aériennes pulmonaires ou encore lors de chirurgie ouvrant l'accès direct ou encore directement à travers d'autres tissus ou organes avec des cathéters, des aiguilles ou des endoscopes adaptés.

Le RGTA peut être également administré en spray sur une surface tissulaire dans le cas d'implantations de cellules isolées ou en feuillets ou encore imprégnés dans des matériaux implantés support pour une colonisation cellulaire existante ou à venir.

Concernant les voies aériennes, l'administration peut être également envisagée par inhalation.

Concernant des muqueuses et/ou des parois du tractus digestif ou des muscles utérins ou des ligaments, la voie rectale ou vaginale a été préférée quand accessible.

Pour le traitement des tissus oculaire, la forme préférée d'administration était par exemple en collyre, par exemple pour le traitement de la cornée, par injection trans-cornéenne par exemple dans le traitement de la membrane de Decemet, dans le traitement de tissus tapissant le fond du globe oculaire, par exemple pour le traitement de défauts de la vision, ou encore par exemple pour le traitement de lésion de tissus liés à l'audition, par exemple pour l'implantation de cellules ciliaires de la cochlée.

Dans les exemples, l'administration ou injection locale peut être unique, quotidienne ou hebdomadaire, la dose étant alors liée à la surface ou volume de la lésion. En particulier, dans les exemples la concentration de RGTA était, de préférence, de 100 microg/mL étant la concentration préférée et le volume utilisé choisi de manière à recouvrir la surface lésée ou imprégner le volume de la lésion. Ainsi, l'injection péri-lésionnelle dans le tissu ou l'organe ou dans le site de 0,1 à 0,5 mL de RGTA a permis d'imprégner un tissu dans un volume de 5 à 100 fois supérieur. Dans l'application de surface ou en spray, le RGTA pénétrant par adsorption, le recouvrement de la zone lésée était suffisant. Ainsi trois à quatre « pulvérisations » de 140 mL chacune à 5 cm de distance de la lésion ont été suffisantes pour imprégner une surface de 10 cm², alternativement quelques millilitres d'une crème ou d'un gel ou pommade peuvent s'étaler sur la lésion ou à proximité et assurer ainsi un accès au RGTA. Les formes galéniques des crèmes, pommades, gels, laits, mousses, émulsions, pates poudres etc... sont celles connues de l'homme de l'art et de préférences choisies pour leurs propriétés hydrophiles et hydratantes compatibles avec des polysaccharides par exemple l'acide Hyaluronique.

Dans les exemples, un autre mode d'administration du RGTA utilisé était celui de imprégnation des tissus, par exemple pour le cas de greffes de tissu ou d'organe comme le rein, foie, coeur, poumon, peau, cornée, tympan, muscles, nerfs, tendons, ligaments, os, vaisseaux ou intestins, colons tant dans les zones d'anastomoses que dans des implants, vessie, etc. sans que cette liste soit exhaustive. Dans ce mode de réalisation qui peut être utilisé à tous tissus ou greffons, l'organe à greffer qui peut être imprégné, par exemple soit en le plongeant dans une solution de RGTA, soit par perfusion, soit par spray ou toute autre méthode connue de l'homme du métier. Avantageusement, l'imprégnation des tissus telle que mentionnée ci-dessus permet avantageusement de recréer le microenvironnement tissulaire. En effet, avantageusement RGTA se fixe spécifiquement sur les sites « heparanes binding » disponibles après la lésion et induits par le prélèvement du greffon ou la préparation du receveur de la greffe. Avantageusement cette fixation permet de récréer une niche qui favorise la colonisation par les cellules. Les concentrations préférées de RGTA étaient de 0,01 à 100 microgramme (mg) par mL. La durée d'imprégnation était courte car quelques minutes suffisent. Avantageusement, une imprégnation de quelques heures ou jour(s) peut permettre de simplifier la procédure car le RGTA peut être ajouté aux solutions de conservation et se révéler comme agent protecteur et anti apoptotique comme cela est décrit dans l'art antérieur (Barritault D., Caruelle J-P. BIP121532, « POLYMERES BIOCOMPATIBLES, LEUR PROCEDE DE PREPARATION ET LES COMPOSITIONS LES CONTENANT » ; et Yue X-L, Lehri S, Li P, Barbier-Chassefière V, Petit V, Huang Q-F, Albanese P, Barritault D, Caruelle J-P, Papy-Garcia D and Morin C. Insights on a new path of pre-mitochondrial apoptosis regulation by a glycosaminoglycan mimetic.; Yue X-L et al, Cell Death and Differentiation, 2009, 1-12). Les tissus ont été alors exposés à des solutions ou suspensions enrichies le cas échéant en cellules d'une spécificité voulue ceci avant ou après l'implantation. A cette technique peuvent être ajouté des extraits ou lysats plaquettaires enrichies en facteurs de croissance.

Les cellules autologues ont été ajoutées avec le greffon avant ou après l'implantation permettant avantageusement de faciliter la colonisation de zones de jonctions hôte greffon et favoriser la prise de greffe. Cette méthode permet une prise de greffe, évite des nécroses et augmente la récupération fonctionnelle.

Dans les exemples, les extraits plaquettaires ont été administrés seuls après avoir administré le RGTA ou avec le RGTA. Dans ce cas le mélange a été réalisé avec ratio RGTA/extrait PRP de manière le lysat des plaquettes obtenus à partir de 10⁹ plaquettes mise en suspension dans 1 mL de sérum physiologique soit mis en présence de thrombine ou à 100 mg de RGTA. A l'issu de la dégranulation des plaquettes la solution a été centrifugé à basse vitesse. Le surnageant contenant les facteurs plaquettaires et le RGTA ont été ensuite administrés sur le site lésionnel.

Dans d'autre mode de réalisation, les RGTA seuls ou mélangés avec des extraits plaquettaires (PRP) ou facteurs isolés ont été injectés avec les cellules thérapeutiques.

Dans les exemples, l'administration du RGTA a été effectuée au plus tard au moment de la première administration des cellules, ou de préférence quelques heures ou jours avant. Ainsi dans le cas de prises orales de RGTA nous avons observé qu'une ou plusieurs prise quotidienne une semaine avant la thérapie cellulaire augmentait le résultat final de régénération tissulaire, la dose préférée étant alors de boire 25 mL du RGTA OTR4120 à 100 mg/mL matin à jeun et le soir avant de se coucher pendant une semaine au moins. Lors d'une injection IV ou locale du RGTA, la thérapie cellulaire pouvait être mise en oeuvre dès l'heure qui suivait l'administration de RGTA.

Dans les exemples décrits ci dessous, les cellules sont autologues et administrées de préférence le même jour que celui de leur prélèvement.

### Exemple 2 : Exemple de traitement d'une lésion tissulaire avec l'administration de RGTA et de Cellules Souches Mésenchymateuses (CSM) dans un modèle murin.

Dans le présent exemple, les souris utilisées étaient 70 souris femelles de 10 semaines (C57/BL6) Charles River divisées en 7 groupes de 10 souris et correspondent à un modèle de souris reconnu dans l'état de la technique.

Une plaie cutanée a été réalisée sur le dos des souris à l'aide d'un emporte-pièce de 6 mm de diamètre (utilisé pour des biopsies en clinique) et la plaie a été ensuite laissée à l'air libre. 4 plaies ont été réalisées sur chaque animal.

Le polymère utilisé était l'OTR4120 et a été administré par injection sous-cutanée (SC) à raison de 25 microL (µL) d'une solution à 100 µL en deux points (diamétralement opposés). Les Cellules Souches Mésenchymateuses (CSM) provenant de moelles osseuses (tibia) isolé selon les protocoles classique tels ceux décrit dans « A protocol for isolation and culture of mesenchymal stem cells from mouse bone marrow», Soleimani M, Nadri S. Nat Protoc. 2009;4(1):102-6, ont été mises en suspension dans du tampon phosphate salin PBS à une concentration de 1 X 10⁶ cellules par mL et injectées à raison de 2 injections de 50 µL par plaie en deux points (symétriques orthogonale à ceux du RGTA) à différents temps et la plaie a été ensuite mesurée à 3,5,7,10 jours après la blessure.

Lors des expériences, pour chaque souris, plusieurs plaie ont été effectuée et une comparaison a été effectuée entre :
- deux injections diamétralement opposées par rapport à la plaie pour le RGTA,
- deux injections diamétralement opposées par rapport à la plaie pour les CSM, et
- quatre injections aux 4 points cardinaux couplant le RGTA et CSM

Différents groupes de souris ont été définis en fonction de la composition administrée selon les procédés précités et sont détaillés ci-dessous :
- Groupe 1 Témoin (Placebo) : administration de Sérum physiologique dans les 15 minutes suivant la plaie/lésion.
- Groupe 2 Administration de CSM : 1 million de CSM par administration en 2 points symétriques en opposition des sites d'injection du RGTA, 24 heures après la blessure après la blessure.
- Groupe 3 : administration de RGTA après blessure
- Groupe 4: administration de RGTA mélangé avec CSM (co-injecté)
- Groupe 5 : administration de RGTA après la blessure suivie d'administration de CSM 5 minutes après.
- Groupe 6 : administration de RGTA après la blessure suivi d'administration de CSM 6 heures après.
- Groupe 7 : administration de RGTA après la blessure suivi d'administration de CSM 12 heures après.
- Groupe 8 : administration de RGTA après la blessure suivi d'administration de CSM 24 heures après

Le tableau ci-dessous résume les résultats concernant la cinétique de fermeture de la plaie mesurée en % sur chaque animal selon les traitements. Au temps Zéro, la surface de la lésion est par définition 100% pour chaque animal, quant la plaie fermée la valeur est égale à 0%.

**Tableau 1 : évolution de la plaie en fonction du temps**

| **Groupe/jours** | **0** | **3** | **5** | **7** | **10** |
|---|---|---|---|---|---|
| 1-PBS | 100 | 53+/-15 | 29+/-15 | 15+/-10 | 5+/-5 |
| 2-CSM | 100 | 38+/-10 | 20+/-5 | 10+/-5 | 0 |
| 3-RGTA | 100 | 40+/-5 | 25+/-5 | 10+/-5 | 0 |
| 4-RGTA+CSM co-injection | 100 | 35+/-5 | 20+/-5 | 8+/-5 | 0 |
| 5-RGTA+ CSM 5mn | 100 | 38+/-5 | 20+/-5 | 8+/-5 | 0 |
| 6-RGTA+ CSM 6h | 100 | 30+/-5 | 15+/-5 | 5+/-5 | 0 |
| 7-RGTA+ CSM 12h | 100 | 15+/-5 | 5+/-5 | 0 | 0 |
| 8-RGTA+ CSM 24h | 100 | 10+/-5 | 0 | 0 | 0 |

Le tableau 1 démontre que l'administration de CSM et de RGTA permet d'améliorer la réparation tissulaire et en particulier permet d'accélérer significativement la cicatrisation. En particulier, l'administration des cellules 24 heures après le RGTA permet une fermeture de la plaie qui est très supérieure à celle obtenue avec le RGTA seul ou les CSM seules.

Un effet comparable a été obtenu avec des cellules provenant d'adipocytes de souris de la même portée.

### Exemples de l'invention observés en clinique

De nombreux exemples réalisés en clinique mentionnés ci-dessous illustrent les effets de l'invention. Le produit RGTA dans sa forme commerciale OTR4120 ou CACIPLIQ® étant facilement accessible. Par contre, l'objectivation est impossible et seules les observations cliniques documentent les effets de l'invention.

### Exemple 3 : Effet du traitement combiné avec le RGTA et les Cellules Souches Mésenchymateuses (CSM) autologues dans la cicatrisation des plaies chroniques.

Dans ces exemples des patients ayant différents types de plaies chroniques d'étiologie variée comme des ulcères diabétiques, veineux, ischémiques, des escarres, des brûlures et des prises de greffe étaient en échec thérapeutique depuis des mois. Plusieurs traitements avaient été essayés sans succès y compris un traitement local au CACIPLIQ® (RGTA OTR4120) selon les recommandations du fabricant.

Ce clinicien avait aussi l'habitude de combiner la technologie du RGTA avec les extraits plaquettaires ou de réaliser des thérapies cellulaires à partir de CSM autologues obtenues par prélèvement de 5 mL de moelle osseuse du sternum sur le patient consentant. Le prélèvement filtré et enrichit par centrifugation selon les techniques décrites pour les CSM étaient mis en suspension dans 1,5 mL du sérum physiologique et la solution cellulaire injectée dans les bords de la plaie en sous cutanée ainsi que dans le centre de la plaie. En tout une douzaine d'injections de 0,1 mL selon le pourtour de l'ulcère (et selon l'image assimilant l'ulcère au quadrant d'une horloge et en injectant à chaque position des heures). Selon ce clinicien, l'utilisation seule soit du RGTA ou de la thérapie cellulaire ne permettait pas une cicatrisation de la plaie.

Une administration combinée selon l'invention de RGTA et des cellules a été également effectuée. Le CACIPLIQ® a été appliqué localement selon le protocole recommandé pour le produit CACIPLIQ : la solution de 5 mL à 100 µL de CACIPLIQ® (RGTA OTR4120) a été versée sur une compresse de 15x15 cm et la compresse imprégnée a été appliquée 5 minutes sur la plaie bien détergée puis retirée. Les CSM ont été ensuite injectées comme décrit ci-dessus (exemple 1) dans l'heure après l'application du RGTA. Le CACIPLIQ® a été ensuite administré localement à raison de 2 fois par semaine. Pour quelques patients un deuxième prélèvement de moelle osseuse a été réalisé 3 semaines après, et les patients ont été à nouveau traités comme précédemment par une injection de CSM. Le résultat a été très rapide puisque la fermeture de tous les ulcères a été observée en moins de 6 semaines.

Une autre série d'essais clinique a été également réalisé sur d'autres patients, par injection du CACIPLIQ® (RGTA) et puis injection des cellules dans l'heure qui suivait leur prélèvement (le temps de préparer les cellules). Le résultat a été alors encore plus étonnant puisque tous les patients ont pu cicatriser complètement en moins d'un mois. De plus, il a même noté que certains de ces patients ont vu leur plaie se fermer en 15 jours alors qu'elle durait depuis des mois sans signe d'amélioration. Une telle rapidité n'avait jamais observé par ce clinicien, quelque soit le traitement appliqué, notamment lors du traitement avec le CACIPLIQ® seul ou les CSM seules.

Cet exemple démontre donc clairement que la composition selon l'invention permet avantageusement et de manière surprenante de traiter efficacement les lésions tissulaires, notamment les plaies chroniques, et permet avantageusement une accélération important de la cicatrisation.

En outre cet exemple démontre clairement que la composition selon l'invention fournit une nouvelle solution à un problème pour lequel aucune solution n'existait dans l'état de la technique.

En outre, un effet comparable sinon encore meilleur a été observé sur des patients par co-administration du RGTA, de PRP et de cellules. Dans un mode de réalisation préférée, l'administration de RGTA a été suivie d'une injection de PRP puis des cellules, administration qui a été pour des raisons réglementaires effectuée dans le même temps opératoire. le RGTA a été administré seul (1 mL à 100 mg/mL de OTR4120), avec ou séquentiellement du PRP (provenant de lysat de plaquettes à partir d'un prélèvement sanguin du patient par exemple 50 mL), avec ou séquentiellement des cellules autologues mésenchymateuses par exemple, mais sans que cela soit limité, de différentes sources tissulaires.

Dans un autre mode de réalisation, un prélèvement de cellules souches mésenchymateuses a été effectué à partir des tissus adipeux du patient à traiter par liposuccion. Le procédé d'isolement des CSM était identique au procédé décrit dans l'exemple 1 ci-dessus

Dans un autre exemple de traitement utilisant la composition selon l'invention, un patient présentant une plaie profonde recouvrant une partie du dessous du pied incluant le gros orteil et comprenant une zone de nécrose visible sur une bonne moitié du dessous de l'orteil. Une odeur forte de décomposition remplissait la pièce de consultation. Un premier traitement par une thérapie cellulaire par injections de 0,.1 mL en plusieurs points autour de la plaie d'un total de 1 mL de cellules mésenchymateuses provenant directement de la moelle osseuse du sternum du patient a été mis en oeuvre sans succès en outre l'état de la plaie s'était aggravée. Un second traitement utilisant l'administration seule de CACIPLIQ (marque déposée) localement n'a pas permis non plus d'améliorer la plaie et a permis uniquement d'éviter une plus forte dégradation et « maintenir » un état de statut quo sur l'évolution et l'aggravation de la plaie évitant ainsi une amputation immédiate. Suite à ces échecs, une injection de 0,5 mL en 5 points de 0,1 microg.mL⁻¹ à 100 microg.mL⁻¹ du RGTA (CACIPLIQ) au niveau de la jonction des zones encore saine de celle nécrotiques de l'orteil puis une heure après 5 injections de 0,1 mL chacune de cellules provenant directement d'un nouveau prélèvement de 0,5 mL de cellules par ponction au niveau su sternum. Suite à l'administration de la composition selon l'invention une régression de la plaie a été observée, permettant d'éviter toute amputation, allant jusqu'à une guérison complète de la plaie. En particulier, la zone nécrotique a été totalement éliminée puis remplacée par un bourgeonnement provenant des tissus encore sains et contigus à la plaie jusqu'à le recouvrement complet de la plaie. Dans cet exemple une seule séquence d'injection (RGTA puis cellules autologues mésenchymateuses) a suffit pour traiter ce patient et permis la fermeture de sa plaie

### Exemple 4: Effet du traitement combiné avec le RGTA et thérapie cellulaire dans la cicatrisation de tissus osseux.

Dans cet exemple le traitement d'une fracture avec défaut d'union mettant a été réalisé en utilisant la composition selon l'invention.

De même l'art antérieur décrit chez des A savoir une association inefficace et dissuasive de l'administration de RGTA avec une thérapie cellulaire a été réalisé dans l'état de la technique mais n'a permis d'obtenir aucune amélioration des fractures et ceux-ci après plusieurs mois. Les résultats sur des plaies ouvertes permettaient d'obtenir une accélération remarquable du processus cicatriciel.

Des patients ayant une fracture du tibia avec défaut osseux (« non union bone fracture ») n'ayant ni réussi à s'améliorer par application du RGTA seul ni après une l'administration de cellules issues de moelle osseuse selon les protocoles de l'art décrits ci dessus ou celui décrit par (Hernigou P, Homma Y, Flouzat-Lachaniette CH, Poignard A, Chevallier N, Rouard H. Cancer risk is not increased in patients treated for orthopaedic diseases with autologous bone marrow cell concentrate. J Bone Joint Surg Am. 2013;95:2215-21).

Des patients n'ayant ni répondu au traitement par le RGTA seul ni au traitement par des cellules autologues prélevées directement de la moelle osseuse, ont été traités avec la composition selon l'invention. De manière surprenante et inattendue, notamment à la lumière de l'enseignement de l'état de la technique, l'utilisation de la composition selon l'invention, notamment la combinaison de RGTA avec les cellules issues de la moelle osseuse a permis d'enclencher le processus cicatriciel chez ces patients et d'obtenir ce qu'aucun traitement n'avait réussi seul, montrant ainsi l'intérêt de l'invention. En particulier, trois patients en échec thérapeutique ont été traités avec la composition selon l'invention et ont pu bénéficier de ce double traitement. Dans ces cas les cellules issues de moelle osseuse ont été administrées 1h après administration de 1mL de RGTA par injection locale dans la zone de none union et de 1 mL de cellules mésenchymateuses, ponctionnées à partir du sternum du patient et réinjectées dans la zone du défaut osseuses sans autre étape de traitement.

Une combinaison comprenant en outre des extraits plaquettaires a également été pratiquée et donne d'excellents résultats lors de son utilisation entre l'administration du RGTA et des cellules. En particulier, des résultats très positifs ont été obtenus lors de l'administration du RGTA, puis une heure après du PRP puis des cellules.

Dans cet autre exemple, l'effet de la composition selon l'invention sur la préservation osseuse et action sur les nécroses ischémiques de la tête de fémur ont été également étudiés. Un traitement utilisant le RGTA et les cellules ou le RGTA, du PRP et des cellules ont été effectués. Les patients présentant des cas d'ischémies de la tête de fémur au stade précoce avant l'installation d'une nécrose profonde et provoquant des douleurs importantes. Un traitement utilisant le RGTA et les cellules selon l'invention a permis d'éviter la destruction de la tête de fémur et la nécessité de procéder à des actes chirurgicaux afin d'implanter une prothèse. Le traitement consistait en l'injection locale de 1 mL RGTA OTR4131 à 10 microg.mL⁻¹ dans la zone ischémique péri-osseuse proche de la lésion suivi 30 minutes après de l'injection locale de 5 mL de cellules issues de moelle osseuse autologues prélevées de la fosse iliaque le jour même.

Dans un autre cas un co-traitement avec du PRP a été réalisé, le PRP était préparé à partir d'un prélèvement de 10 mL de sang périphérique selon les procédés usuels et injectés localement quelques minutes avant les cellules du prélèvement. Les résultats obtenus via ces deux traitements ont montrée une préservation de la tête du fémur. Il est à noter que chez plusieurs de ces patients présentant une nécrose des tissus déjà installée, une disparition totale de la nécrose suggérant un processus de régénération et de remplacement des tissus nécrotiques. La combinaison du RGTA et des cellules autologues permet avantageusement d'obtenir cette régénération avec un effet synergique et surprenant, amélioré par l'injection du PRP. Dans cet exemple, l'effet de la composition selon l'invention dans le traitement de destructions osseuses dans le cas d'une ostéonécrose post-infection a été étudié.

La destruction du tissu osseux est une conséquence fréquente et une complication du traitement des plaies chroniques suite à une ostéomyélite d'origine infectieuse bactérienne normalement traitée en longue durée par antibiothérapie. La composition/procédé selon l'invention a été mis en oeuvre et a permis de sauver et de reformer du tissu osseux. L'action synergique du RGTA complétée par la thérapie cellulaire a permis une régénération du tissu osseux lésé comme jamais observée avec le RGTA seul ni la thérapie cellulaire seule. Plusieurs patients ainsi traités par plusieurs injections locales de 0.1 mL de CACIPLIQ (OTR4120 à 100 microg.mL⁻¹) dans la partie de tissue sain autour de la zone nécrotique suivie d'injection de même volume de cellules mésenchymateuses prélevées du sternum du patient sans autre préparation ont permis ainsi de récupérer un tissu osseux avant sa destruction complète, ce qui aurait été impossible sans cette thérapie. Il est à noter que ce traitement était fait en maintenant l'antibiothérapie et qu'il n'a cependant pas été observé une repousse osseuse intégrale, le traitement permettant de sauver voir de récupérer mais pas une repousse ex nihilo d'un os entièrement détruit.

### Exemple 4 : Traitement des lésions articulaires et tendineuses avec un exemple de composition selon l'invention.

L'art antérieur décrit les effets de l'injection de RGTA seul, notamment le OTR4131 dans le traitement des lésions tendineuses et articulaires chez le cheval de sport (Coudry V et al Journal of Equine Veterinary Science, 2014, 34 Pages 1357-1360, David Carnicer, mémoire de recherche « Preliminary report : ultrasonographic evolution of tendon lesions treated with RGTA in horses » école nationale vétérinaire de Maison Alfort), il en est de même de l'utilisation de thérapie cellulaire, notamment de cellules mésenchymateuses prélevés chez le cheval au niveau de la fosse iliaque ou plus couramment du sternum (Pechayre M. et Betizeau C. S0704, congrès AVAC, 2-4 Décembre 2011). Lyon congrès annuel.

### Le PRP a également été utilisé dans les mêmes indications (http://fr.slideshare.net/dvmfun/platelet-rich-plasma-prp-therapy)

Une étude comparative a été effectuée en utilisant des chevaux traités soit en combinant RGTA et les cellules selon l'invention, le RGTA et PRP, RGTA, PRP et thérapie cellulaire par rapport à un traitement avec uniquement des cellules.

Dans cet exemples les chevaux présentaient soit des lésions tendineuses (au niveau de tendon SFD) ou articulaires (ostéochondroses disséquantes, kyste osseux sous-chondraux, lésions du ménisque). Dans tous les cas le traitement combiné RGTA et cellules mésenchymateuses a permis une récupération beaucoup plus rapide des chevaux à la course (reprise à environ 5-6 mois soit un gain de un a deux mois comparé au temps de récupération après traitement au RGTA seul ou un traitement cellulaire seul). Cette récupération étant évaluée au niveau de la récupération à la boiterie, un retour plus rapide à l'entrainement et aux performances antérieures pour presque tous les chevaux traités. Pour ces chevaux le RGTA a été injecté dans un premier temps dans la semaine suivant la lésion tendineuse sous échographie. L'injection de cellules a eu lieu selon les cas, le même jour mais après celle du RGTA (le plus souvent environ 30 minutes à une heure correspondant au temps nécessaire de préparation des cellules). L'injection de RGTA a parfois été suivie quelques minutes après par l'injection de PRP puis de l'injection des cellules autologues.

Les quantités de RGTA, de cellules et de PRP étaient respectivement de 1 mL d'OTR4131, 1,5 mL de lysat de PRP (à partir de 10 mL de sang prélevé et 1 mL de cellules autologues et l'administration du PRP et des cellules étant effectuée une heure après la première injection de RGTA

Les meilleurs résultats ont été obtenus dans le cas de lésions articulaires lorsque les cellules ont été injectées mélangées avec un biomatériau, comme le collagène ou l'acide Hyaluronique ou une combinaison des deux. Dans ces cas ces produits sont de qualité injectable en solution (utilisé en chirurgie plastique pour la ride ou articulaire chez l'homme) ont été directement ajoutés à la solution de cellules avant injection. La solution de cellule étant alors diluée deux fois environ : 1 mL de cellules et 1 mL de solution de biomatériaux de préférence entre 0.1 à 3 mg.mL⁻¹

Dans un autre mode de réalisation une administration en un bolus a été effectuée comprenant soit RGTA et cellules ou RGTA, PRP et cellules. Les résultats obtenus ont montré un traitement efficace et significativement améliorer par rapport à un traitement comprenant l'administration d'un seul des éléments précités. Par ailleurs, des résultats encore plus surprenants ont été obtenus en espaçant l'administration de RGTA et de cellules d'une durée de 30 à 60 minutes à quelques heures (de préférence le même).

Actuellement, le traitement de lésions et souffrances articulaires de patients est effectué par des thérapies cellulaires et notamment par l'injection locale de cellules mésenchymateuses autologues provenant de moelle osseuse ou de tissu adipeux.

Toutefois, ce traitement ne permet pas d'obtenir un traitement efficace et présente des échecs. Aussi, un traitement utilisant la composition selon l'invention via notamment l'administration (injection) de RGTA dans le liquide synovial suivi de l'injection de cellules mésenchymateuses autologues ou d'adipocytes a été pratiqué chez plusieurs patients en échec d'une thérapie cellulaire seule. Pour ce faire, les quantités de RGTA et de cellules étaient respectivement 1 mL à 100 microg.mL⁻¹, de 1 à 5 mL de cellules autologues enrichies en cellules mésenchymateuses adipocytaire par centrifugation et injectées le jour même, en général 30 minutes à une heure après l'injection de RGTA. Le timing d'administration d'injection des cellules a été réalisé dans l'heure suivant l'injection de RGTA ou une injection de deux solutions, l'une de RGTA immédiatement suivie d'une injection des cellules ou l'administration simultanée du RGTA et des cellules via une solution unique.

L'addition d'acide Hyaluronique après l'injection de RGTA mais avant ou avec l'injection de cellules ainsi que la combinaison avec d'autres biomatériaux comme les collagènes type 2 ont donné des résultats encore meilleurs chez certains patients.

Suite à ce traitement et de manière surprenante, les lésions et souffrances associées ont été résorbées permettant avantageusement aux patients de remarcher progressivement sans avoir besoin de l'implantation d'une prothèse de genoux et sans douleur.

En particuliers, chez une dizaine de patients atteints de lésions ostéochondrales, très handicapés dans leurs mouvements et par la douleur, ayant préalablement été traités sans succès avec des cellules ni avec le RGTA seul. Suite au traitement précité, huit d'entre eux ont pu récupérer une capacité motrice grâce au double traitement RGTA puis cellules autologues, démontrant clairement les avantages surprenants et inattendus de la composition selon l'invention.

### Exemple 5 : Traitement des lésions des tissus du système digestif avec une composition selon l'invention

L'art antérieur décrit les propriétés du RGTA sur les muqueuses du tractus digestif, buccales (Morvan et al., Am J Pathol. 2004 Feb; 164(2):739-46), gingivales (Escartin et al., FASEB J. 2003 Apr; 17(6):644-51), au niveau d'ulcérations au niveau de l'estomac ou des intestins (Meddahi et al., J Biomed Mater Res. 2002, 60(3):497-501); ainsi que leur capacité a réduire les fibroses en agissant au niveau des synthèses de collagènes sur des cellules isolées, normale ou irradiées (Alexakis C. et al., FASEB J. 2001 Jul; 15(9):1546-54) ainsi que sur des biopsies provenant de tissus de patients atteints de la maladie de Crohn (Alexakis C. et al., Gut. 2004 Jan; 53(1):85-90). Toutefois, aucun effet des RGTA sur des lésions des tissus du système digestif, en particulier concernant une éventuelle réparation des lésions, n'a été observé ou identifié.

Dans cet exemple, un traitement utilisant la composition selon l'invention à savoir un RGTA et des cellules a été appliquée sur un patient atteint de la maladie de Crohn et souffrant d'une fistule périnéale depuis plusieurs années. Dans cet exemple, le RGTA utilisé à une concentration de 100 microg.mL⁻¹ de 0,5 mL a été injecté à proximité de la fistule (0.1 mL /par injection et en 3 sites) suivi dans les 30 minutes de l'injection de la fraction enrichie en cellules provenant d'un prélèvement de 10 mL de liposuccion) (0,1 mL par injection en deux ou trois sites de cellules)

L'administration de la composition selon l'invention a permis de fermer la fistule péri-anale alors que ni le RGTA seul, ni l'administration de ces mêmes cellules n'avait permis d'obtenir cette fermeture et guérison.

Un deuxième patient ayant subit une ablation chirurgicale d'un carcinome épidermoïde très proche du sphincter suivi d'une chimio-radiothérapie ayant perdu la fonction de son sphincter a pu bénéficier d'une injection locale de RGTA OTR4120 (0.1.mL à 100 microg.mL⁻¹) en plusieurs sites suivie après 30 minutes d'une injection locale de cellules adipocytes autologues (100 microL/injection provenant d'un volume initial de liposuccion de 10 mL) . Suite à cette administration une colonisation rapide des cellules du site lésionnel a été observée permettant au patient une récupération fonctionnelle du sphincter ce qui ne semblait impossible.

### Exemple 6: Traitement de lésions tissulaires comprenant l'administration d'une composition selon l'invention:

Dans ces exemples, il n'y a pas eu d'objectivation de l'effet additif combinant RGTA et thérapie cellulaire, car chaque cas est isolé et unique ce qui ne permet pas une objectivation du ou des effets. Le seul critère observé et susceptible d'être étudié est l'amélioration ou non du patient après traitement.

Dans cet exemple, l'effet de la composition selon l'invention dans la régénération pulmonaire a été étudié. Des patients atteints de lésions des muqueuses pulmonaires provoquées par une exposition à des vapeurs toxiques d'incendies ont inhalé du RGTA OTR4120 mis dans un vaporisateur et les cellules autologues adipocytaires injectées le jour même une heure après l'inhalation. Le vaporisateur contenait une solution à 100 microg.mL⁻¹ d'OTR4120, et l'inhalation était pendant 10 minutes permettant l'inhalation d'environ 5 mL. Dans la demi heure suivante des cellules autologues prélevées le même jour par liposuccion (sans concentration) étaient injectées (5 mL par voie IV) Cette administration a permis de manière surprenante et inattendue une récupération rapide et fonctionnelle du patient, à savoir une respiration améliorée alors que le patient traité était en échec thérapeutique de plusieurs mois.

Dans cet exemple, l'effet de la composition selon l'invention sur des tissus lésé suite à la pose d'un stent a été observé. Pour ce faire une administration de RGTA par voie orale a été faite le jour de la mise en place du stent et suivit après 24h de l'administration de cellules autologues adipocytaires par voie I.V. (provenant d'une liposuccion de 50 mL) La prise orale de RGTA OTR4120 était de 50 mL d'une solution à 100 microg.mL⁻¹ a été maintenue pendant 1 mois. L'injection de cellules répétée à 10 J et 20 Jours

Une recolonisation de la zone lésée après pose du stent a été observé mais non documentée montrant de manière surprenante une ré-endothélisation de la zone d'implantation du stent, ce qui n'avait jamais été observé avec le RGTA seul ou les cellules seules.

Dans cet exemple, l'effet de la composition selon l'invention sur des tissus lésés ici un muscle irradié accidentellement et ayant subit une perte fonctionnelle sa fonction. Pour ce faire une administration orale de RGTA à savoir 25 mL à 100 mg/mL pendant deux jours suivit de l'administration dans la zone du muscle irradiée de cellules, à savoir 5 injections de 1 mL d'une solution de cellules provenant de 50 mL de prélèvement de moelle osseuses prélevée de la fosse iliaque le même jour puis enrichie par centrifugation et suspendues en 5 mL de sérum physiologique. Suite à l'administration une évaluation de son activité fonctionnelle a été effectuée. De manière surprenante et inattendue, le traitement a permis une récupération fonctionnelle motrice ce qui semblait impossible.

Dans cet exemple, l'effet de la composition selon l'invention sur la ré-épithélialisation de cornée a été étudié. Pour ce faire un ensemencement le jour du prélèvement de cellules prélevées à partir des muqueuses buccales du patient sur des cornées préalablement traitées localement par deux gouttes de RGTA par cornée a été effectué. Ce traitement a permis d'obtenir une réparation du tissu lésé et en particulier l'effet a été considérablement augmenté par l'administration du CACICOL seul.

Enfin, une évaluation de l'effet d'un exemple de composition selon l'invention sur une lésion de la moelle épinière a été également effectuée. Un patient âgé de 20 ans présentait une dégradation de la fonction motrice suite à une lésion récente de la moelle épinière le rendant paralysé des membres inférieurs. Un traitement sur trois jours utilisant un exemple de composition selon l'invention pour laquelle les quantités de RGTA et de cellules étaient respectivement 25 mL/jour de RGTA par voie orale d'une solution à 100 mg/mL d'OTR4120 et de l'injection locale (dans la zone périphérique de la lésion de la moelle épinière) de 1 mL en 4 points de cellules provenant d'une ponction de 50 mL de moelle osseuse provenant de la fosse iliaque. Cette injection a été renouvelle à 10 et 20 jours alors que la prise quotidienne orale de RGTA était maintenue pendant une durée de deux mois.

De manière inattendue et très positive, le patient a commencé à récupérer une petite fonction neuro-motrice après les trois premiers jours de jours de traitement et progressivement une amélioration sensible jusqu'à une récupération motrice.

## Revendications

1. Composition pharmaceutique ou dermatologique pour son application comme médicament pour la prévention et/ou le traitement de lésions tissulaires, ladite composition comprenant
- un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et
R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
- une cellule eucaryote.

2. Composition pharmaceutique ou dermatologique pour son application comme médicament pour la prévention et/ou le traitement de lésions tissulaires, ladite composition comprenant
- un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et
R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
- un extrait et/ou lysat plaquettaire

3. Composition pharmaceutique ou dermatologique pour son application comme médicament pour la prévention et/ou le traitement de lésions tissulaires, ladite composition comprenant
- un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
- Au moins un facteur de croissance.

4. Composition selon l'une quelconque des revendications 1 à 3, dans laquelle les monomères A identiques ou différents sont choisis parmi les sucres, les esters, les alcools, les acides aminés, les nucléotides, les acides nucléiques, les protéines ou des dérivés de ceux-ci.

5. Composition selon l'une quelconque des revendications 1 à 4, dans laquelle les monomères A identiques ou différents sont choisis parmi les sucres ou dérivés de ceux-ci.

6. Composition selon l'une quelconque des revendications 1 à 5, dans laquelle un maximum de 20% des monomères A sont indépendamment des monomères de formule suivante : 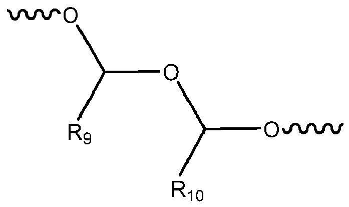 dans laquelle R₉ et R₁₀ représente indépendamment un atome d'oxygène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué, un groupement de formule -R₁₁-(X)ₙ-R₁₂ dans laquelle R₁₁ représente une chaine carbonée aliphatique en C₁ à C₄, éventuellement ramifiée et/ou insaturée, X représente un hétéroatome choisi parmi l'oxygène et l'azote, n est un entier compris de 1 à 4 et R₁₂ est un atome d'hydrogène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué.

7. Composition selon l'une quelconque des revendications 1 à 6, dans laquelle le nombre de monomère « a » est tel que la masse desdits polymères de formule (I) est supérieure à 2000 daltons.

8. Composition selon l'une quelconque des revendications 1 à 7, dans laquelle le taux de substitution « x » est compris entre 20 et 150%.

9. Composition selon l'une quelconque des revendications précédentes, dans laquelle le taux de substitution « y » est compris entre 30 et 150%.

10. Composition selon l'une quelconque des revendications précédentes, **caractérisée en ce que** le radical R est choisi parmi un groupement alkyle, allyle, aryle, linéaires ou ramifiés.

11. Composition selon l'une quelconque des revendications précédentes, dans laquelle que ledit polymère biocompatible comprend en outre des groupements chimiques fonctionnels Z, différents de X et Y, capables de conférer audit polymère des propriétés biologiques ou physicochimiques supplémentaires.

12. Composition selon la revendication 11, dans laquelle le taux de substitution « z » de l'ensemble des monomères A par des groupements Z est compris de 0 à 50%.

13. Composition selon la revendication 11 ou 12, dans laquelle le groupement Z est une substance capable de conférer auxdits polymères une meilleure solubilité ou lipophilie.

14. Composition selon la revendication 13, dans laquelle les groupements Z sont identiques ou différents et sont choisis dans le groupe comprenant des acides aminés, des acides gras, des alcools gras, des céramides, ou des dérivés de ceux-ci, ou encore des séquences nucléotidiques d'adressage.

15. Composition selon l'une quelconque des revendications 6 à 14 dans laquelle les groupements R₉ et R₁₀ sont indépendamment et éventuellement substitués par un groupement Z.

16. Composition selon la revendication 1 ou l'une quelconque des revendications 4 à 15 lorsqu'elles dépendent de la revendication 1 dans laquelle la cellule eucaryote est choisie dans le groupe comprenant des cellules eucaryotes adultes ou embryonnaires, des cellules de moelle osseuse, des cellules du tissu adipeux.

17. Composition la revendication 1 ou l'une quelconque des revendications 4 à 16 lorsqu'elles dépendent de la revendication 1 dans laquelle ledit polymère biocompatible est administré dans le traitement de lésions tissulaires par :
- par voie intraveineuse ou par voie intramusculaire à une dose comprise de 0,1 à 5 mg/kg de poids corporel, ou
- par injection locale à une dose comprise de 1 à 100 microgramme par millilitre,
- par voie orale en 2 à 5 prises égales par jour à hauteur d'un total quotidien de 10 microg à 5 mg/kg de poids corporel
- par voie sublinguale à jeun d'une solution concentrée aqueuse de 1 à 100mg/mL,
- par administration en aérosol ou spray nasal d'une solution,
et dans laquelle ladite cellule eucaryote est administrée dans le traitement de lésions tissulaires par injection dans un délai de 5 minutes à 24h après la première prise dudit polymère biocompatible.

18. Composition selon la revendication 1 ou l'une quelconque des revendications 4 à 17 lorsqu'elles ne dépendent pas de la revendication 2 ou 3 dans laquelle la composition comprend au moins un facteur de croissance choisi dans le groupe comprenant le facteur de croissance des fibroblastes (FGF), le facteur de croissance vasculaire endothéliale (VEGF), le facteur de croissance dérivé des plaquettes (PGDF) ou un mélange de ceux-ci.

19. Composition selon la revendication 1 ou l'une quelconque des revendications 4 à 18 lorsqu'elles ne dépendent pas de la revendication 2 ou 3 dans laquelle la composition comprend un extrait plaquettaire et/ou lysat plaquettaire.

20. Composition selon l'une quelconque des revendications précédentes dans laquelle les lésions tissulaires sont choisis parmi les lésions du système locomoteur, les lésions vasculaire, les lésions cardiaque, les lésions du système nerveux central de préférence à l'exception des lésions ischémique du système nerveux central, les lésions du système nerveux périphériques, les lésions des systèmes sensoriels, les lésions du système nerveux respiratoire, les lésions du tractus digestif, les lésions des tissus osseux, les lésions du tissu pancréatique, les lésions du tissu hépatique, les lésions du tissu rénale, les lésions des voies urinaires et/ou les lésions du tissu vaginale, de préférence à l'exception des lésions ischémiques des membres inférieurs.

21. Kit pharmaceutique pour la prévention et/ou le traitement de lésions tissulaires comprenant :
**i.** un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et
R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
**ii.** une cellule eucaryote.

22. Kit pharmaceutique selon la revendication 16 dans lequel le polymère biocompatible est utilisable
- par voie intraveineuse ou par voie intramusculaire a une dose comprise de 0,1 à 5 mg/kg de poids corporel, ou
- par voie orale en 2 à 5 prises égales par jour à hauteur d'un total quotidien de 1,5 à 500 mg,
- par voie intracrânienne à une dose comprise de 0,001 à 1 mg.mL-1
- par voie sublinguale à jeun d'une solution concentrée aqueuse de 1 à 100mg.mL⁻¹,
- par voie aérienne par aspersion d'une solution comprenant de 0,1 à 5 mg/kg de poids corporel dudit polymère,
et dans laquelle ladite cellule eucaryote est utilisable pour injection dans un délai de 5 minutes à 24h après la première administration dudit polymère biocompatible.

23. Kit pharmaceutique selon la revendication ou dans lequel le polymère biocompatible et/ou ladite cellule sont administrés sur une période d'un jour à 3 mois.

24. Kit pharmaceutique selon l'une quelconque des revendications à, dans lequel le polymère biocompatible et/ou ladite cellule dans lequel l'administration est quotidienne, biquotidienne ou hebdomadaire.

25. Utilisation d'une composition pharmaceutique comprenant
**i**.un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et
R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
**ii.** une cellule eucaryote
pour la fabrication d'un médicament pour le traitement de lésions tissulaires.

26. Utilisation d'une composition pharmaceutique comprenant
**i.**un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
**ii.** un extrait et/ou lysat plaquettaire
pour la fabrication d'un médicament pour le traitement de lésions tissulaires.

27. Utilisation d'une composition pharmaceutique comprenant **i.**un polymère biocompatible de formule générale (I) suivante
AaXxYy (I)
dans laquelle :
A représente un monomère,
X représente un groupement R₁COOR₂,
Y représente un groupement O ou N-sulfonate et répondant à l'une des formules suivante -R₃OSO3R₄, -R₅NSO3R₆ dans lesquelles :
R₁, R₃ et R₅ représentent indépendamment une chaîne hydrocarbonée aliphatique, éventuellement ramifiée et/ou insaturée et éventuellement qui contient un ou plusieurs cycles aromatiques et
R₂, R₄ et R₆ représentent indépendamment un atome d'hydrogène ou un cation,
a représente le nombre de monomères,
x représente le taux de substitution des monomères A par des groupements X,
y représente le taux de substitution des monomères A par des groupements Y, et
**ii.** au moins un facteur de croissance
pour la fabrication d'un médicament pour le traitement de lésions tissulaires.
